# EUROPEAN PATENT SPECIFICATION

(11) **EP 3 347 870 B1**
(45) Date of publication and mention of the grant of the patent: **01.04.2026**
(21) Application number: 16845182.1
(22) Date of filing: 09.09.2016
(51) Int. Cl.: H04L 9/40, H04W 12/02, G06F 21/62, H04W 12/06, G16H 10/60, G16H 10/65, G06F 21/36, G06F 21/42

(54) **SECURE REAL-TIME HEALTH RECORD EXCHANGE**
SICHERER ECHTZEITAUSTAUSCH VON KRANKENAKTEN
ÉCHANGE SÉCURISÉ DE DOSSIER DE SANTÉ EN TEMPS RÉEL

(30) Priority: 09.09.2015 US 201562215834 P; 29.06.2016 US 201662356519 P; 08.09.2016 US 201615260312
(43) Date of publication of application: 18.07.2018
(73) Proprietor: Humetrix, Del Mar, CA 92014 (US)
(72) Inventor: EXPERTON, Bettina, Del Mar, CA 92014 (US); BURROW, Christopher, Del Mar, CA 92014 (US); MICKELSEN, Stephen, Del Mar, CA 92014 (US)
(74) Representative: Purdylucey Intellectual Property
(86) International application number: PCT/US2016/051093
(87) International publication number: WO 2017/044841

(56) References cited:
- WO-A1-2014/206795
- US-A1- 2009 186 322
- US-A1- 2014 316 812
- US-A1- 2014 316 812
- US-A1- 2015 100 348
- US-A1- 2015 169 551

## Description

### CROSS-REFERENCE TO RELATED APPLICATIONS

This application claims the benefit of U.S. Utility Application Serial No. 15/260,312 filed in the U.S. Patent Office on September 8, 2016 which claims priority to both U.S. Provisional Application Serial No. 62/215,834 filed in the U.S. Patent Office on September 9, 2015, and of U.S. Provisional Application Serial No. 62/356,519 filed in the U.S. Patent Office on June 29, 2016.

### BACKGROUND

### Field

The present invention relates generally to electronic healthcare records and more particularly to access and exchange of electronic healthcare records using mobile computing devices.

### Background

In today's healthcare environment individuals typically receive healthcare from multiple healthcare providers and often at multiple locations. Healthcare providers commonly lack accurate and up-to-date information regarding the care previously received by a patient from other providers. In order to deliver optimum, coordinated healthcare and most cost-effective healthcare to their patients, healthcare providers need to have ready access to an up to date medical history of their patients wherever they have received care, and the ability to exchange their most recent clinical findings and treatment plans to other healthcare providers who will be caring for their patients next. US Patent Publication No. US 2014/0316812 describes a method of streamlining the intake of registration of a patient in a healthcare provider facility by providing a secure, online database via a web-based portal for a patient to enter and store the patient's private health information data. A unique readable scanning code is assigned to the data so that a healthcare provider facility can read the code and access the patient's health information data.

### SUMMARY

In an aspect of the disclosure, an electronic medical records access system as set out in the appended claims comprises a portable computing device uniquely associated with one of a plurality of users. The portable computing device may be configured to execute an agent that authenticates an identification of the one user associated with the portable computing device. The portable computing device may be configured to execute an agent that automatically retrieves information corresponding to the one user from at least one electronic healthcare records system using the identification to access the at least one electronic healthcare records system. The portable computing device may be configured to execute an agent that electronically delivers a portion of the information to a healthcare provider. Delivery may be effected through a network server.

The portable computing device may authenticate one or more of a user and a recipient of records and other information using a Bluetooth connection, a wireless network or by optical exchange of information that provides a communication path that is separate and distinct from the networking path used to deliver records. In one example, a QRC may be presented to a healthcare provider, whereby the QRC includes a network location of the records and cryptographic keys necessary to decrypt the records once retrieved from the network location. The portable computing device may directly deliver the portion of the information electronically using a Bluetooth connection, a wireless network or by another method of communication.

In an aspect of the disclosure, the portable computing device comprises a wireless telephone, a smart phone, a wearable computing device, a smart watch, a health or fitness tracker, eyewear, and/or a tablet computer. The portable computing device may retrieve the information from the at least one electronic healthcare records system using a cellular wireless telephone network. A portion of the information may be delivered to a computing device, such as a desktop or portable computing device operated by the healthcare provider. A portion of the information may be delivered using a server communicatively coupled to the portable computing devices associated with the one user and operated by the healthcare provider. A portion of the information may be encrypted.

In an aspect of the disclosure, the agent combines the retrieved information with other information retrieved from the at least one electronic healthcare records system to obtain combined information. Other information may comprise electronic health records of the user that are maintained by the portable computing device. The electronic health records maintained by the portable computing device may be encrypted using encryption keys uniquely associated with the one user.

In an aspect of the disclosure, a portion of the combined information or single health record delivered to the healthcare provider is selected based on consent of the record holder that may be expressly given or inferred from a request to transfer files to the provider, where the record holder has chosen to transfer these files. The consent may be based on an identification of the user. The identification of the user may be authenticated using a biometric measurement.

In an aspect of the disclosure, an electronic device comprising one or more processors and non-transient storage maintains data and instructions configured to cause one or more processors of a computing system to authenticate an identification of a user uniquely associated with the electronic device, automatically retrieve information corresponding to the user from at least one electronic healthcare records system using the identification to access the at least one electronic healthcare records system, and electronically deliver a portion of the information to a healthcare provider.

The electronic device may be adapted to be communicatively coupled to the computing system. A portion of the information may be delivered to a computing device operated by the healthcare provider. The computing device of the healthcare provider may be a portable computing device and may comprise a wireless telephone, a smart phone, a wearable computing device, a smart watch, a health or fitness tracker, eyewear, and/or a tablet computer. A portion of the information may be delivered using a server communicatively coupled to the portable computing device. A portion of the information may be encrypted.

In an aspect of the disclosure, retrieved information may be combined with other information retrieved from the at least one electronic healthcare records system to obtain a report or combined record. The other information retrieved from electronic healthcare records systems may comprise electronic health records of the user that are maintained by the portable computing device.

### BRIEF DESCRIPTION OF THE DRAWINGS

FIG. 1 illustrates an example of a hardware implementation for an apparatus employing a processing system.
FIG. 2 illustrates an example of an electronic records delivery system according to certain aspects described herein.
FIG. 3 illustrates flow of electronic health records between a patient and physicians in accordance with certain aspects described herein.
FIG. 4 illustrates a first example of proximity exchange between client and provider devices according to certain aspects described herein.
FIG. 5 illustrates certain aspects of a proximity exchange initiated in response to a medical emergency in accordance with certain aspects described herein.
FIG. 6 illustrates a second example of proximity exchange between client and provider devices according to certain aspects described herein.
FIG. 7 illustrates an example of the delivery of medical records to users of systems deployed according to certain aspects described herein.
FIG. 8 is a block diagram illustrating an example of an apparatus employing a processing circuit that may be adapted according to certain aspects disclosed herein.
FIG. 9 includes flowcharts illustrating certain aspects of health record exchanges in accordance with certain aspects described herein.
FIG. 10 illustrates a first example of a hardware implementation for an apparatus employing a processing system configured to perform certain functions according to certain aspects described herein.
FIG. 11 illustrates a second example of a hardware implementation for an apparatus employing a processing system configured to perform certain functions according to certain aspects described herein.
FIG. 12 includes a flowchart illustrating certain aspects of an automated health record exchange involving according to certain aspects described herein.
FIG. 13 illustrates a third example of a hardware implementation for an apparatus employing a processing system configured to perform certain functions according to certain aspects described herein.

### DETAILED DESCRIPTION

The detailed description set forth below in connection with the appended drawings is intended as a description of various configurations and is not intended to represent the only configurations in which the concepts described herein may be practiced. The detailed description includes specific details for the purpose of providing a thorough understanding of various concepts. However, it will be apparent to those skilled in the art that these concepts may be practiced without these specific details. In some instances, well known structures and components are shown in block diagram form in order to avoid obscuring such concepts.

Several aspects of records management systems will now be presented with reference to various apparatus and methods. These apparatus and methods will be described in the following detailed description and illustrated in the accompanying drawing by various blocks, modules, components, circuits, steps, processes, algorithms, etc. (collectively referred to as "elements"). These elements may be implemented using electronic hardware, computer software, or any combination thereof. Whether such elements are implemented as hardware or software depends upon the particular application and design constraints imposed on the overall system.

By way of example, an element, or any portion of an element, or any combination of elements may be implemented with a "processing system" that includes one or more processors. Examples of processors include microprocessors, microcontrollers, digital signal processors (DSPs), field programmable gate arrays (FPGAs), programmable logic devices (PLDs), state machines, gated logic, discrete hardware circuits, and other suitable hardware configured to perform the various functionality described throughout this disclosure. One or more processors in the processing system may execute software. Software shall be construed broadly to mean instructions, instruction sets, code, code segments, program code, programs, subprograms, software modules, applications, software applications, software packages, routines, subroutines, objects, executables, threads of execution, procedures, functions, etc., whether referred to as software, firmware, middleware, microcode, hardware description language, or otherwise. The software may reside on a computer-readable medium. A computer-readable medium may include, by way of example, a magnetic storage device (e.g., hard disk, floppy disk, magnetic strip), an optical disk (e.g., compact disk (CD), digital versatile disk (DVD)), a smart card, a flash memory device (e.g., card, stick, key drive), Near Field Communications (NFC) token, random access memory (RAM), read only memory (ROM), programmable ROM (PROM), erasable PROM (EPROM), electrically erasable PROM (EEPROM), a register, a removable disk, a carrier wave, a transmission line, and any other suitable medium for storing or transmitting software. The computer-readable medium may be resident in the processing system, external to the processing system, or distributed across multiple entities including the processing system. Computer-readable medium may be embodied in a computer-program product. By way of example, a computer-program product may include a computer-readable medium in packaging materials. Those skilled in the art will recognize how best to implement the described functionality presented throughout this disclosure depending on the particular application and the overall design constraints imposed on the overall system.

### Example Of An Apparatus

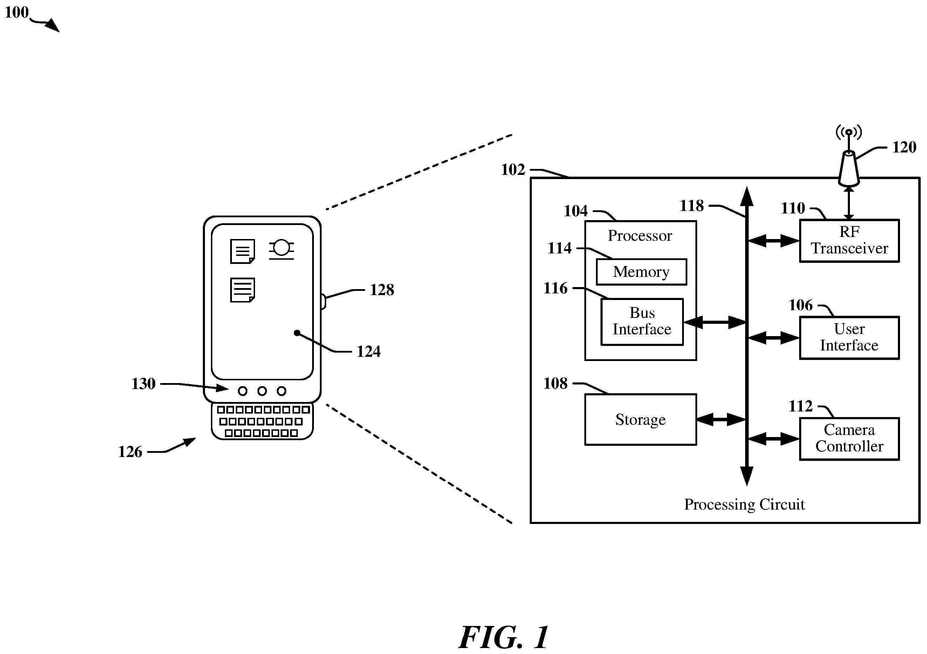

FIG. 1 illustrates an example of an apparatus 100 that may be adapted in accordance with certain aspects disclosed herein. The apparatus 100 may be embodied in a processing circuit 102, which may comprise one or more integrated circuit (IC) devices 104, 106, 108, 110, and/or 112. In this example, the processing circuit 102 may be implemented with a bus architecture, represented generally by the bus 118. The bus 118 may include any number of interconnecting buses and bridges depending on the specific application of the processing circuit 102 and the overall design constraints. The bus 118 links together various circuits and/or devices 104, 106, 108, 110, and/or 112, including one or more processors, represented generally by the processing device 104, a user-interface device 106, computer-readable storage media, represented generally by the storage device 108, one or more communication interfaces, represented generally by the radio frequency (RF) transceiver 110, and an imaging interface, represented generally by the camera device 112. The bus 118 may also link various other circuits such as timing sources, peripherals, voltage regulators, and power management circuits, which are well known in the art, and therefore, will not be described any further. The processing circuit 102 may include a bus interface 116 that provides an interface between the bus 118 and the processing circuit 102. In some instances, the bus interface 116 controls and provides access between multiple devices 104, 106, 108, 110, and/or 112. In some embodiments, bus interface 116 may be an integral part of the processing circuit 102. In some embodiments, the bus interface 116 may interface a processing system with standards-defined bus, such as a universal serial bus (USB), or the like, that permits external peripherals to be coupled to the apparatus 100.

An RF transceiver 110 or other transceiver may provide a means for communicating with various other apparatus over a transmission medium. Another type of transceiver may be employed to provide a proprietary wired interface or a wired interface compliant or consistent with a standard such as universal serial bus (USB), FireWire, Ethernet, Serial Advanced Technology Attachment (SATA), etc. The RF transceiver 110 may provide a wireless interface and transmit and receive radio signals through an antenna 120 using a proprietary or standardized signaling protocol such as IEEE 802.11, WiFi, WiMax, CDMA, WCDMA, Bluetooth, etc. In one example, the RF transceiver 110 and an antenna 120 may enable the apparatus 100 to communicate as a radio frequency identification device (RFID) device. Other transceivers may enable optical, infrared and other communications. Depending upon the nature of the apparatus, a user interface 106 (e.g., keypad, display, speaker, microphone, joystick) may also be provided.

The processing device 104 is responsible for managing the bus 118 and general processing, including the execution of software stored on the computer-readable medium 108. The software, when executed by the processing device 104, causes the processing circuit 102 to perform the various functions described *infra* for any particular apparatus. The computer-readable medium 108 may also be used for storing data that is manipulated by the processing device 104 when executing software.

### Electronic Records Including Electronic Health Records

The various concepts presented throughout this disclosure may be implemented using a device that is configured to interface and/or interact with broad variety of telecommunication systems, network architectures, and communication standards.

Various aspects of the present disclosure relate to an example involving electronic health records. The scope of the invention is not limited to electronic health records and various aspects of the invention may relate to the management and access of other types of records, including legal records, financial records, employment records, and so on. For example, certain aspects of the invention are applicable to point-of-sale authorization and identification of the parties to a transaction. In another example, certain aspects of the invention may enable secure transactions and exchange of information between clients and financial institutions. For simplicity of description, however, examples involving electronic health records are used throughout this disclosure.

In the example of electronic health records, portable computing devices may be used to authenticate a patient and/or a healthcare provider to enable and/or authorize and exchange of the electronic health records. The patient may elect to push electronic healthcare records to the healthcare provider. The healthcare provider may elect to push updates and/or new records to the patient. Healthcare records may include images, such as radiographic images initially captured through the use of radiography, magnetic resonance imaging (MRI), computerized tomography (CT-Scan or CATSCAN), ultrasonic imaging, or other imaging processes. Records and updates may be pushed over local networks using a Bluetooth connection, a wireless network or by optical exchange of information that provides a communication path that can be separate and distinct from the networking path used to deliver records. In one example, a quick response code (QRC) may be presented to a healthcare provider, whereby the QRC includes information that can be used to identify a network location of the records, cryptographic keys necessary to decrypt the records once retrieved from the network location, and other information.

The portable computing devices may directly deliver the portion of the information electronically using a Bluetooth connection, a wireless network or by an intermediate network server, or by any other method of electronic or wireless communication. Exchange of records and other information between the patient and the provider may be effected using multiple communications channels or links. In one example, a first channel may provide information that includes a network address of the records and corresponding cryptographic keys necessary to extract the records, while a second channel may be used to deliver the encrypted records and/or cryptographic keys. The first channel may be implemented using a camera or optical scanner to read an encoded optical image, such as a QRC or other barcode.

### Examples Of Networks Configured To Transfer Electronic Health Records

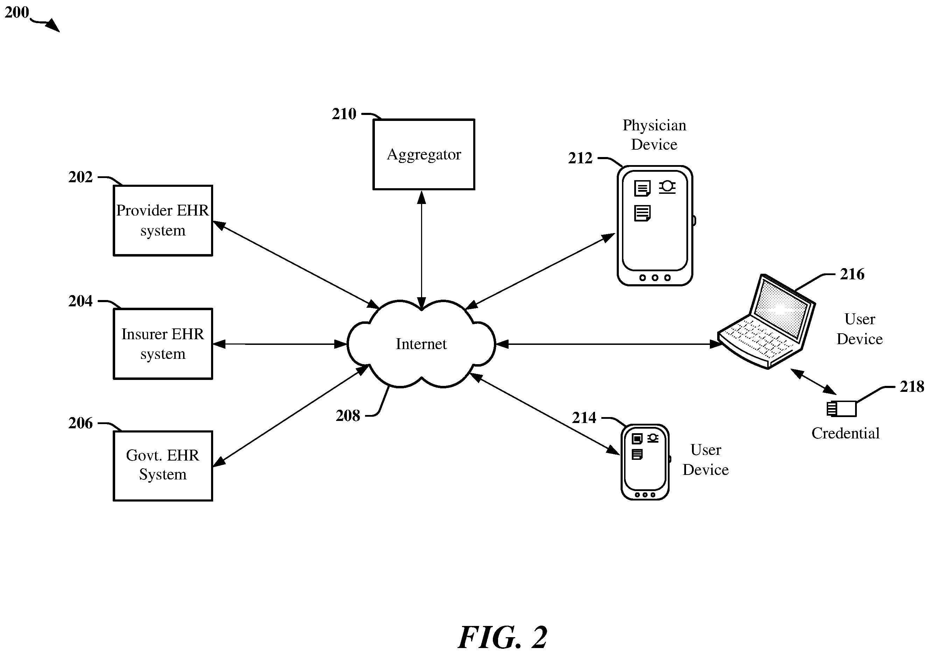

FIG. 2 illustrates a simplified example of a system 200 adapted according to certain aspects of the invention. Electronic Health Records (EHRs) may be maintained in various physical locations and/or on EHR systems 202, 204, and 206 operated by a plurality of different parties including healthcare provider EHR systems 202, payer EHR systems such as insurers and/or EHR systems 206 operated by government entities. In one example, records maintained on EHR systems 202, 204, and 206 may include duplicate information maintained in two or more of the EHR systems 202, 204, and 206. In other examples, at least some EHR information may be aggregated, accumulated, and/or maintained in a single EHR system 202, 204 or 206.

A user may access records through a client device 214 or 216, such as a smart phone, a tablet computing device, a notebook computer, a wearable computing device, a smart watch, a health or fitness tracker, eyewear, or other suitable mobile device. In some instances, the user may access records through an appliance that incorporates or is controlled by a computing system or other processing device. The user may be a service provider. The user may be an individual record owner who may be a client or patient of a provider system and/or a client or an individual insured by an insurer, or an agent of the record owner. In certain circumstances, the user may be an emergency responder acting on behalf of a debilitated, injured or otherwise incapacitated individual record owner. In many instances, the record owner is a patient who receives healthcare services in multiple locations and/or from multiple healthcare providers. Healthcare providers may include one or more of a primary care provider (physician), a physician specialist, an emergency responder and a pharmacy. The patient may be insured by a private or public health insurance plan. Each of these different healthcare entities may maintain separate and distinct electronic health records for the patient.

A client device 214, 216 may be adapted or configured to enable access to personal electronic health records. In some examples, an application or agent may be installed and/or downloaded to the client device 214, 216 to enable access to personal electronic health records that are maintained on one or more centralized databases corresponding to the EHR systems 202, 204 and 206. The user may access electronic health records related to a transaction or the provision of healthcare services to a patient, and the records accessed may comprise personal health records, such as medical records and insurance records, which may be remotely located on centralized databases embodied in EHR systems 202, 240, and 206 operated by a service provider, insurer or other entity.

In one example, databases maintained by one or more EHR systems 202, 204, and 206 may be accessed through a network 208. The network 208 may be implemented using a wireless network, a cellular access network, the Internet and/or one or more private network, etc. In certain embodiments, a record owner can access EHR systems or databases individually to retrieve records related to a specific activity, service, and/or provider. In some embodiments, the record owner may identify a set of EHR systems or databases to be accessed and aggregated, combined, collated, or merged to obtain a combined set of EHR records and/or a report identifying relevant or available EHRs. In some embodiments, the record user can specify a type of record to be accessed, regardless of which EHR systems or databases maintain such records or types of records. In some embodiments, a record owner can generate a combined individual record for immediate access and use by the user, or for delivery to a healthcare provider such as a physician. Records may be delivered to the physician through the physician's personal computing device or a computing device owned or operated by a healthcare provider (e.g., the physician device 212). The record owner may produce a combined record on-demand (on-the-fly), or may provide access to a combined individual record that is maintained by, or on behalf of the record owner and which is updated automatically and/or periodically. In some embodiments, the record owner may authorize and/or enable a provider to access EHRs from a single source, from multiple sources, and/or from an aggregator 210. In some embodiments, a record owner may authorize and/or enable a provider to access certain types of records, regardless of the location of those records.

In the example illustrated in FIG. 2, the individual records may be delivered to a physician device 212, such as a tablet computer or smart phone, although the combined individual record may also be delivered to a server or other computer of an EHR system 202, 204 or 206. In some embodiments, the record owner may cause a server or other network device (e.g., an aggregator 210) to deliver the combined individual record sourced from one or more EHR systems 202, 204, or 206 to a physician device 212 or other computing device, such as a desktop computer. In one example, the aggregator 210 may be used to provide individual records when a record owner does not have access to a client device 214 capable of producing and delivering the individual record or when the record owner's device (client device 214, 216, 218) cannot connect to the physician device 212 or EHR systems 202, 204, or 206.

Identification and authentication information may be maintained on a client device 214, 216 to permit the record owner to access each of EHR systems 202, 204, and 206. The maintenance and control of the identification and authentication information by the record owner can reduce overall system complexity because a single command and identification process at the record owner's device (e.g., client device 214 or 216) can initiate automatic access to relevant records on the EHR systems 202, 204, 206 and/or to relevant records provided by an aggregator 210. For example, an agent installed on the client device 214, 216 may be configured to identify and authenticate the user of the client device 214, 216 through password, challenge words, a biometric scan and/or other means of authentication known in the art. In some examples, authentication may be confirmed by a trusted third party device or service provider. Authentication information may be provided to each of the EHR systems 202, 204, and 206 and/or the aggregator 210 to enable access to the EHR information related to the record owner. In one example, the client device 214, 216 of the record owner may supply the authentication information. In another example, the trusted third party device or service provider may provide the authentication information.

The process of authentication and/or point of origin of the request may be recorded and may be used to prove consent of a record holder to a transfer of records to a provider. In some embodiments, a request from a user to transfer records may be considered or configured to include consent of the record owner, based on prior identification and/or authentication of the identity of the user as the record holder. The record owner may be presented with a request to confirm a transfer request. The request for confirmation may include a request for identification and/or a request to authenticate the identity of the recipient of the transfer request. In some embodiments, the user may configure the type of transfer to be performed for each request. For example, consent may be limited to a subset of the owner's EHR record. In some embodiments, the record owner may configure a default specification of the types of record that can be transferred to one or more service providers. Authenticated requests to transfer information and acknowledgements of such requests, as well as acknowledgements of delivery and/or acceptance of a requested EHR may be logged at the client device 214 or 216, the physician device 212, a physician management system, one of the record holder's EHR systems 202, 204, 206, and/or an aggregator 210. The user may authorize and/or initiate an access to EHRs through the facilities of a service provider.

The user may prepare a combined EHR report or may store a set of EHR information from a variety of sources on a mobile device or on a storage device. Locally maintained information is typically encrypted. The record holder may transfer a portion or all of locally maintained information to a healthcare provider when seeking healthcare services. The user may also access certain records on-line from home to check on his insurance status, medical appointments, to see prescription refill status or to communicate by e-mail with his physicians.

Certain embodiments provide an interface to multiple electronic health records for both users and service providers. A user may provide authorization that enables a service provider to access some or all of the user's combined records. A first provider may, at the user's discretion, access the user's individual EHRs maintained by a second provider where the second provider may be physically located at a different healthcare facility. In one example, a physician may directly and easily access all of the user records necessary to obtain a current view of the user's complete medical history, insurance eligibility status, and other information. Moreover, medical practitioners can directly access the user's records in order to update the user's health information.

When transferring records, user identification information may be authenticated using any combination of a user ID, password, challenge question and biometric information. Typically, the transfer is made contingent upon a two-way identification of a record holder and a healthcare provider. In-person identification may be made using direct sight. Additionally, both parties' portable devices may establish a connection that is confirmed by both the record holder and the healthcare provider. In one example, the connection may comprise a session secured using encryption keys that are exchanged between the users. The encryption keys may be used to encrypt and decrypt information transmitted between the devices of the users. In some embodiments, the transfer may be restricted to proximately located devices. In one example, the record holder may initiate contact by selecting a physician's tablet computer from a list of devices within Bluetooth range, or within the same WiFi domain. The physician typically accepts the connection before the transfer is initiated.

In certain embodiments, records may not be exchanged without first obtaining a positive identification of the recipient. When the record holder and the healthcare provider are located in different physical locations, information identifying a physical location may be provided by one or more of the record holder and the healthcare provider. The identification of a physical location may be made using a global positioning system, location information provided by a wireless network and from other sources, including triangulation by a cellular network. For example, certain wireless network telecommunications services can provide accurate positional information based on triangulation and/or certain signaling characteristics of mobile devices. In some embodiments, an authentication service may be used to verify identity of a record holder and a healthcare provider, and the record holder and the healthcare provider may be connected when the authentication service confirms identity of the parties, even when the parties are located in different physical locations.

In certain embodiments, user devices of a record holder and a healthcare provider may be incompatible and may not be capable of direct connection. For example, and Android-based device may not be able to connect securely with a tablet computer that operates using a different operating system. When incompatible devices are used, a gateway may be employed to facilitate the connection of the devices. The gateway may provide extended handshake services that identify both devices and establish a secure link between the devices. The gateway may be provided using a local or network server and/or a cloud service.

In certain embodiments, global positioning technology may be used to confirm specific locations and/or proximity of the record holder and provider devices. In some embodiments, radio access technologies such as fourth generation long term evolution (4G LTE) may include location services that can be used to determine proximity or physical location information.

General purpose computing devices 216, such as a notebook or desktop computer, may also be used to access medical records, even where the computer 216 does not belong to the record owner. Record owner may provide an electronic credential 218 that, when read and used by computer 216, enables automatic access of combined individual records. Electronic credential 218 may comprise a hand-held device with a non-transitory memory and an embedded microprocessor or other programmable device. The electronic credentials may comprise a smart card, a USB flash drive, and radio-frequency identification (RFID) device, a Near Field Communication (NFC) token, web-enabled phones, etc. The electronic credentials may be embodied in an identification card or other format easily stored and secured by the user.

In certain embodiments, access to the user's EHR information may be obtained by presenting the electronic credential 218 to a computing device (e.g., the client device 214 or 216), whereby the computing device can establish a wired or wireless connection with the electronic credential 218 that enables an exchange of data. The electronic credential 218 may comprise a small portable device issued by an insurer, a government agency, a primary healthcare provider system, etc. The electronic credential 218 may comprise a memory that maintains information including a personal identifier, a unique identifier assigned to the individual, an EHR locator address, login information, and/or other identifying information. The user may use the electronic credential 218 to access one or more EHR systems 202, 204, and 206 through a client device 214 or 216, such as a personal computer (PC), tablet computer, smart phone, wearable computing device (e.g., a smart watch, a health or fitness tracker, eyewear, etc.), or other suitably equipped processing device. In one example, the electronic credential 218 comprises a flash drive, a smart card, or a device that can connect wirelessly to the client device 214 or 216. The user may present the electronic credential 218 to the client device 214 or 216 in a manner appropriate to allow the electronic credential 218 to exchange information with the client device 214 or 216, whereby the client device 214 or 216 may automatically access and login to one or more EHR systems 202, 204, and 206 using the record owner's identification. The user may have access to the EHR systems 202, 204, and 206 for automated and simultaneous real-time access to medical records maintained therein. In one example, an agent or other application software embedded in the electronic credential 218, or accessed through a network 208 using information stored on the electronic credential 218, may be downloaded to the client device 214 or 216 to enable harvesting of selected data from the different EHR systems 202, 204, and 206 and generate an on-the-fly summary record for a physician to view and use.

Certain embodiments enable automated access to multiple data sources. In one example, an electronic credential 218 comprises an encrypted "electronic keychain" that may be maintained as a knowledgebase that comprises identification and lists of sources of health related information for an individual. The knowledgebase can include both the Internet address as well as identification and other credentials needed to enable access to the data. Typically the health information is maintained by a plurality of healthcare providers or practitioners, and information may be accessible through repositories or databases, including insurance databases and healthcare record portals.

An electronic credential 218 may comprise a device that includes a combination of hardware and software that can encrypt and decrypt information stored on the electronic credential 218. The electronic credential 218 may be embodied in intelligent electronic devices (e.g., devices having at least a programmable controller), such as a universal serial device, a smart phone, a wearable computing device, a smart watch, a health or fitness tracker, eyewear, a PC and a tablet computer. The electronic device may have sufficient processing capacity and storage to operate as a self-contained EHR access portal.

In certain embodiments, an on-the-fly summary of health information can be provided at a medical provider facility, or other location. Information provided by an electronic keychain may be used to initiate access and retrieval of information sourced from multiple EHR systems 202, 204, and 206. Information provided by the electronic keychain may include one or more agents or applications that may compile multiple electronic health records into a single summary form. The summary form may be provided in a standardized format, such as continuity of care record ("CCR"), a continuity of care document ("CCD"), and other suitable formats. In some embodiments, compiled health records may be presented in a consistent summary format regardless of the format used by the originating source. Accordingly, information provided or accessed through the electronic keychain may include templates and conversion modules that can be used to filter and reformat EHR information sourced from a variety of EHR systems 202, 204, and 206.

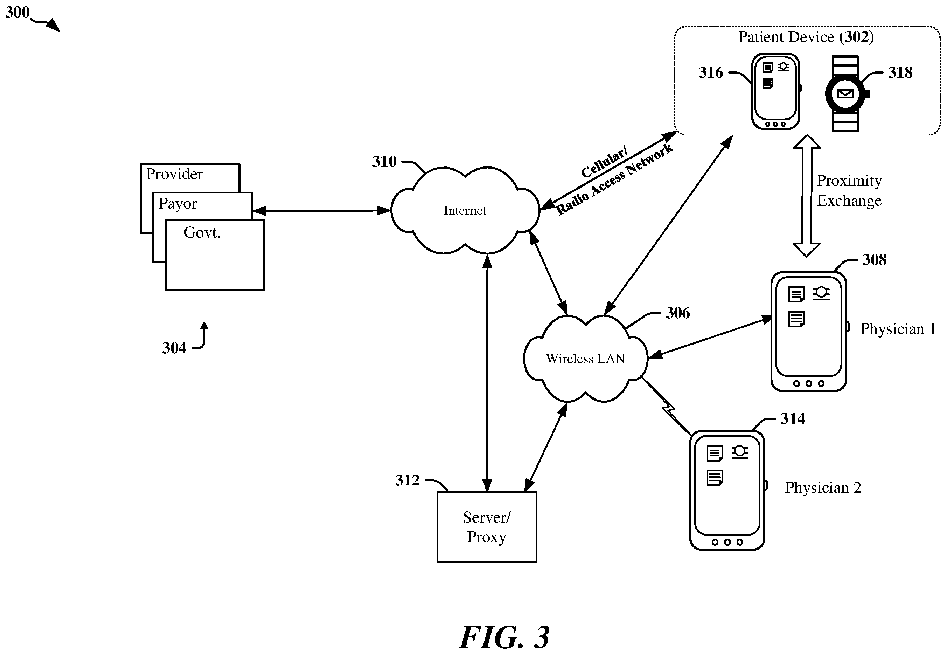

FIG. 3 is a diagram 300 depicting an example of a network architecture that can support the various data flows involved in transactions related to the transfer of EHR records in accordance with certain aspects disclosed herein. In a first scenario, a record owner may use a personal portable computing device (patient device 302) to directly transfer, or push, a combined record to a first provider device 308. For example, a patient visiting a physician's office may wish to provide updated records to the attending physician. In one example, the patient may initiate an agent or other application on a patient device 302 to perform the transfer, where the patient device may be a smartphone 316 and/or a smartwatch 318. The user may be required to provide identifying information, such as a username, a password, an answer to a challenge question and/or the user may be required to provide biometric information, such as a fingerprint, iris scan or submit to facial recognition process or the like. The user may typically select which records should be provided to the physician.

Upon authentication, the agent may determine if a single or combined record is maintained on the patient device 302 and whether such record is current. The agent may request records from one or more healthcare providers, insurers, government agency, public payer or other source of EHR information (shown generally at 304). Having combined or updated the individual record or records, the agent may cause the patient device 302 to push a single record or a set of combined records to the physician device 308 for immediate display. An application or agent on the physician device 308 may be manually initiated to receive the pushed information. In some embodiments, the physician device 308 may be adapted to respond to the push by opening an application or agent to receive or display the records upon receipt of a request for connection from patient device 302.

In certain embodiments, the physician may update records or retrieve other records on the physician device 308 and cause the updated or other records to be transmitted to the patient device 302. The patient device 302 may then provide the new or updated records to one or more of the EHR systems 304 or to another provider's computing device. In some embodiments, the physician may provide medical information to the patient device 302. For example, the physician may receive an X-Ray image on device 308 and may transfer the image to the patient device 302. In another example, the physician may cause device 308 to transmit information to the patient device that provides access to instructional or educational information to the patient device 302, including information on medications, dosage regimens and general information, such as educational information related to a medical condition.

The patient device 302 and the physician device 308 may communicate using any available network or communication method, including WiFi, cellular communications, Bluetooth, IEEE 802.15 (Zigbee), and other short range wireless communications. In certain embodiments, communication between devices 302 and 308 may be restricted to the use of short range communications methods to enhance security. For example, the use of a Bluetooth link between physician device 308 and patient device 302 may limit communications range to a single room, allowing both the physician and patient to verify that communication is properly established between devices 302 and 308 and to ensure that the patient's privacy can be better protected. In certain embodiments, a patient may wish to transfer records to a physician who is not physically present using a wireless LAN 306 located in a medical facility and/or through the Internet 310 where the physician and patient are geographically remote from one another. In such cases, the patient and physician may establish a video conference connection to verify identities and to confirm that communication is properly established between the respective devices 302 and 308.

In a second scenario depicted in FIG. 3, a proxy server 312 may act as a proxy between patient device 302 and a second physician device 314. As described for the first scenario, the patient may initiate a records transfer using the patient device 302. In certain embodiments, the proxy server 312 may provide one or more services, including user identification and authentication services as well as record aggregation services when the patient device 302 is not configured or adaptable to perform such functions. For example, a record owner may provide an electronic credential 218 (see FIG. 2) to a general purpose computing device 216, whereby the electronic credential 218 causes the computer 216 to transmit a request for service to the proxy server 312. In one example, the proxy server 312 may provide a web page to the computing device 216 in order to permit the patient to initiate a request that may be executed by the proxy server 312 on behalf of the patient.

In another example, the patient device 302 and the second physician 314 may be unable to communicate directly. A proxy server 312 may be configured to perform a gateway or routing function that permits exchange of information between the respective devices 302 and 314 through a wide area network (such as the Internet) or a local area network, for example. The devices 302 and 314 may be unable to establish direct Bluetooth or WiFi connections with one another due to security settings of the second physician device 314 and/or the wireless LAN 306. In one example, the intermediary server or proxy server 312 may provide a gateway function through a WiFi network (e.g., the wireless LAN 306) when the patient device 302 is connected to a different domain (e.g., a guest domain), while the second physician device 314 is connected via a secured private domain of the wireless LAN 306.

### Proximity Exchanges

In certain embodiments, proximity may be defined as closeness in both place and time. A proximity exchange may occur when real-time communication of health records and/or health information occurs between the patient device 302 and the physician device 308 while the devices 302 and 308 are in physical proximity with each other and the users can identify each other by direct sight. In certain embodiments, proximity exchange may be used to communicate health records and/or health information from a patient device 302 to a physician device 308 over a local wireless network during a specific time period. In certain embodiments, proximity exchange may be used to initiate the push of health records and/or health information to the physician device 308 during a specific time period, whereby the proximity exchange is used for authentications and/or to provide information necessary for secure transmission of the health records and/or health information to the physician device 308.

The time period associated with a proximity exchange may be defined by a starting time when the communicating parties can identify each other by direct sight, either on a physical line-of-sight or by viewing each other through a video communication session. Typically, the two people exchanging information may be expected to be together in the same room during the proximity exchange. As an example, a patient with a smartphone 316 and/or a smartwatch 318 can send his health records to his doctor who is waiting with her tablet (or other physician device 308) in the same examining room. In another example, a doctor can send the patient treatment instructions at the end of the visit, and/or provide literature related to a diagnosis made by the doctor. In addition to having proximity of space (i.e. being in the same room) the patient and the doctor may also have proximity of time. Each party is expecting the communication to occur more or less immediately, for instance at the time when the physician is asking her patient about his medical history. In some embodiments, virtual identification can be made when the parties can see each other's face through a video link. In some instances, video linked devices 302, 308, and/or 314 may be adapted to perform facial recognition, iris scanning, fingerprint scanning or other biometric scanning when direct and/or indirect visual identification cannot be made by the parties. In some embodiments, visual recognition or a biometric alternative is required to permit access to the EHR information to be exchanged between the parties.

Proximity exchange can provide improved security for EHR exchanges. Proximity exchanges typically limit an EHR exchange by location and time, and an EHR exchange may be initiated by an EHR owner in the presence of recipient of the EHR exchange. Moreover, the opportunity to complete an EHR exchange may be restricted in time, such that EHR exchange must be initiated within a predefined time. An EHR exchange may be characterized as a one-time push, whereby the push cannot be repeated and each push requires separate authorization by the record owner.

### Proximity Exchange Examples

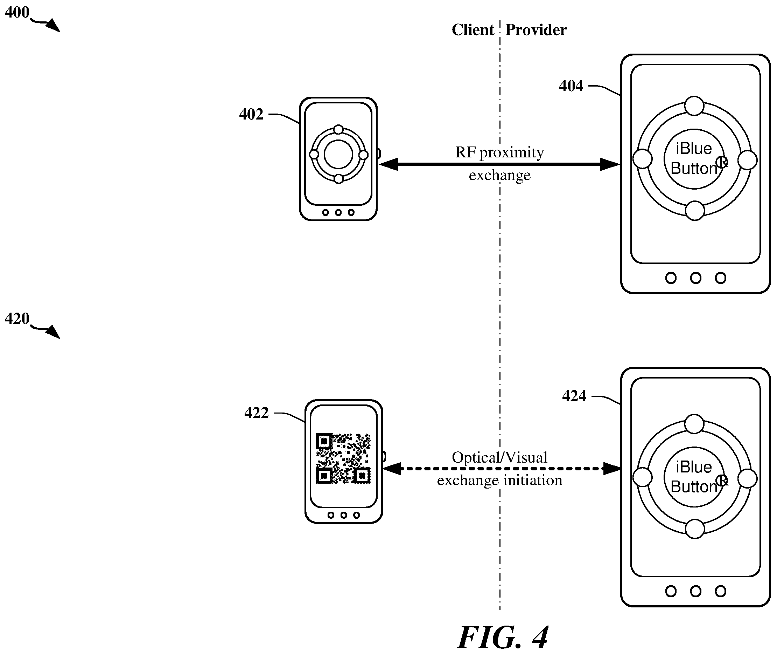

FIG. 4 includes examples 400 and 420 of proximity exchange that illustrate improved security when, for example, an EHR exchange is initiated between a patient (client) and healthcare provider. In some instances, proximity exchange may require that both parties to the exchange are in the same location and/or can visually or audibly confirm the identity of the other party. Proximity exchanges may employ limited range electronic communications, such as Bluetooth and other short range RF communication technologies, NFC interactions, RFID, optical communications, ad hoc connections, and so on. However, proximity exchange may also include exchanges that occur within the same building and/or wireless network segment or cell, when an affirmative identification of the parties can be made.

In one example 400, a proximity exchange is enabled when two devices 402, 404 and/or 422, 424 are in direct communication and proximately located. The client device 402 may be a smartphone, tablet, a wearable computing device, a smart watch, a health or fitness tracker, eyewear, media player, appliance, or other suitable device. The client device 402 may be equipped with an agent or other downloaded application that is configured to provide access to EHR information associated with the client. The provider device 404 may be a personal computer, notebook, smartphone, tablet, media player, or other suitable device and may be equipped with an agent or downloaded application that provides provider access to one or more systems, including a practice management system, EHR systems 202, 204, 206 (see FIG. 2), and/or other systems such as an aggregator 210. The client, having decided to push EHR records to provider device 404, may interact with the agent or application on client device 402 to authenticate patient identity and initiate transfer. EHR exchange may be performed directly by client device 402, or indirectly through a proxy or other server. The client device 402 may transmit information wirelessly to the provider device 404, whereby the information may cause the agent or application on the provider device 404 to initiate receipt and acceptance of the EHR records. Typically, the client/patient may confirm that the push is targeting the provider device 404 based on a personal interaction with the provider and/or confirmation provided through interactions between the client device 402 and the provider device 404.

In another example 420, an EHR exchange can be secured even if the client device 422 is not in communication with the provider device 424 through a networking connection. For example, both devices 422 and 424 may be independently connected to the Internet, but may be unable to connect by Bluetooth or by local networks such as a WiFi network, NFC or Zigbee. In some instances, the client and/or the provider may choose not to use wireless network authentication, or may be prohibited from using wireless network authentications. In some of these examples, secure EHR exchange may be provided through the use of an authentication process employing a combination of a wired network, and an authentication process that involves a proximate exchange of information.

In the depicted example 420, an EHR exchange may be secured by optically exchanging authentication information between two devices 422 and 424. The client device 422 may be a smartphone, tablet, media player, appliance or other suitable device that is equipped with a camera or optical reader. An agent or application installed on the client 422 provides access to EHR information associated with the client. The provider device 424 may be a personal computer, notebook, smartphone, tablet, media player, or other suitable device and may be equipped with a camera or optical reader. An agent or application installed on device 424 provides provider access to one or more systems, including a practice management system, EHR systems 202, 204, 206 (see FIG. 2), and/or other systems such as an aggregator 210.

The client, having decided to push EHR records to provider device 424, may interact with the agent or application on client device 422 to authenticate patient identity and initiate transfer. In order to authenticate the parties to the EHR exchange, the client device 422 may be configured to present an optical image on a display. The provider may capture the image through a camera integral to the provider device 424 or attached to the provider device 424. The image can be decoded to retrieve an encryption key, a file location, and/or other information necessary to authenticate the provider device 424 during the EHR exchange. The provider device 424 may be configured to generate and display an encoded image that can be captured by a camera of the client device 422 and decoded with a response or acknowledgement. In some embodiments, the exchange may be initiated at the provider device 424, which may create and display an image that is captured and used by client device 422 for identification purposes and to permit EHR records to be encrypted and/or directed to the provider device 424 during the EHR exchange. Any suitable type of encoded image may be used, including a barcode such as a QRC.

### Proximity Exchange In Medical Emergencies

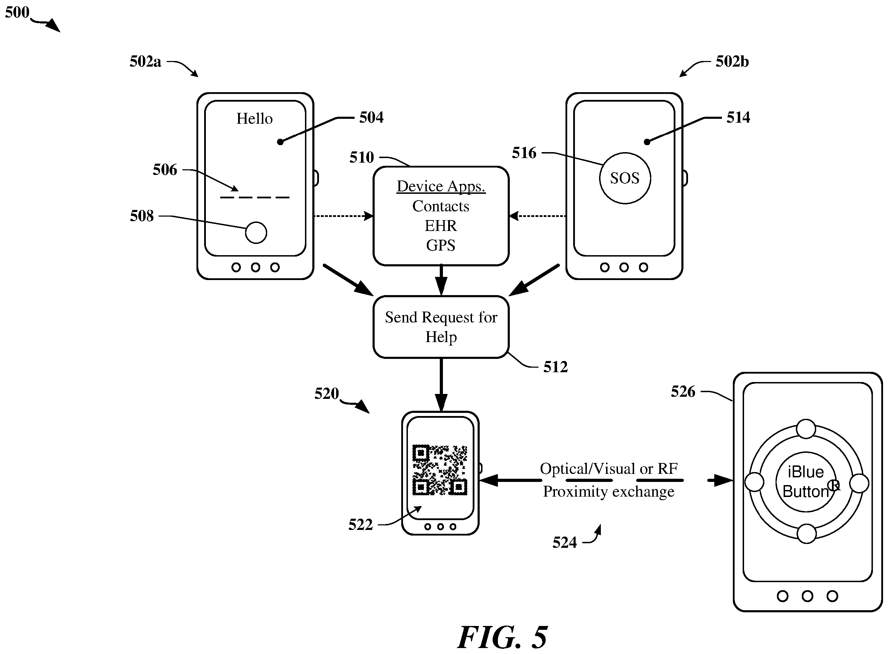

FIG. 5 illustrates certain aspects of a proximity exchange initiated in response to a medical emergency, potential medical emergency or other event. The exchange may be initiated using a user device 520, which may be a cellular phone, a smart phone, a session initiation protocol (SIP) phone, a laptop, a notebook, a netbook, a smartbook, a personal digital assistant (PDA), a satellite radio, a global positioning system (GPS) device, a multimedia device, a video device, a digital audio player (e.g., MP3 player), a camera, a game console, an entertainment device, a vehicle component, or a wearable computing device (e.g., a smart watch, a health or fitness tracker, eyewear, etc.). The user device may be adapted or configured to receive an alarm or an alert and to request assistance using one or more of a telephonic call or an SMS message, an MMS message or through a transmission of information according to a standards-defined or proprietary protocol.

In certain embodiments, an EHR exchange may be secured by optically providing authentication information from the user device 520 to a first responder device 526, without receipt of an express consent to the transaction by the client/patient at the time the transaction occurs. Such exchange may occur, for example, between the user device 520 and a first responder device 526 operated by a first responder paramedic, physician, nurse or other provider who is responding to an emergency. Accordingly, the user device 520 of an incapacitated client may provide authorization that enables a first responder or other provider to access client medical records without initiation of the transaction or transfer by the client.

The user device 520 may be configured to display, or provide access to a first-responder encoded image (FREI) in different modes of operation 502a, 502b. In one example, the user device 520 may be configured to display, or provide access to the FREI when the user device is in a mode of operation 502a where a home screen or login screen is displayed. In another example, the user device 520 may be configured to display, or provide access to the FREI when the user device is in a mode of operation 502b where a home screen is displayed. The FREI 522 may comprise an authentication QRC that can be displayed on a screen provided when a third party wishes to call an emergency service without logging onto the user device 520. In another example, an icon, link and/or reduced-size version of the FREI 522 may be provided on a screen accessible by the first responder or other medical provider, such that activation of the icon, link and/or reduced-size FREI 522 may display a full-size version of the FREI 522 for scanning. In another example, first responders and other pre-authorized providers may enter information including a first-responder identification (FRID) at an initial logon screen of the user device 502 in order to access an authentication code, whereby the FRID may be universal to all user devices 502 subscribed to a wireless network system, and where the FRID may be changed on a regular basis. In some instances, the ID may be entered through a network, where the first responder device 526 initiates a call to the user device 502.

In certain embodiments, the FREI 522 may be generated by the client and printed for use by first responders should an emergency occur. The printed FREI 522 may be updated from time to time and may include sufficient information that provides a first responder with authorization to access the client's medical records using the provider mobile device 526. As described herein, the first responder may be required to provide identifying and authenticating information before access to the medical records is granted. The request sent to the server to fetch the client's medical records may contain provider mobile device 526 specific information, such as a unique device ID (UDID) on a tablet computer, for example. Accordingly, access to medical records may be restricted to pre-authorized devices based on identifying information of the devices.

The FREI 522 may include information that identifies the client and provides access to some or all of the medical records of the client. Access may be limited to certain records which may be determined or expected to be relevant, necessary or desirable during an emergency involving the client. The client may provide advance authorization to permit access to the relevant medical records and the client may specify which records can be made available. In some instances, the client may provide graduated authorization that permits a first-responder access to detailed medical records necessary or useful for treating the client under foreseeable emergency conditions, and that permits public access to certain records or information that may be disclosed without compromising the client's privacy interests. An example of publicly accessible records may include "Medic-Alert" style information which identifies known medical conditions of the client that could render the client incapacitated, and/or that identify allergies suffered by the client, including drug allergies or resistance or reactions to drugs that could cause distress to the client if administered during an emergency situation.

In certain embodiments, the FREI 522 may provide sufficient information that allows an authorized first responder or other provider to access client medical records subject to authentication of the identity of the first responder or provider. The first-responder may transmit a request that includes the FREI 522 or information extracted from the FREI 522, together with identifying information that can prove the identity of the first responder and/or indicate levels of authorization to access medical records. In some instances, the first responder may be challenged by an authentication server or application to provide additional authenticating information. The first responder may be challenged if requests for certain types of client medical records are requested. Interactions with first responders and client medical records may be logged and cross-referenced to the first responder or other provider.

In one example of an embodiment, an application such as the iBlueButton^{®} may be installed on the user device 502. The application may configure the user device 502 to provide a QRC on certain display screens of the user device 502, including the lock screen for example. A first responder or provider may scan the QRC using an iBlueButton Pro^{®} application ("ProApp.") installed on a first responder device 526 in order to facilitate transfer of the client medical records to the ProApp. during an emergency, even if the client is physically unable to authorize the transfer. The QRC may be visible when the user device 502 is not in active use. According to certain aspects of the invention, the QRC may be decoded only by authorized versions of the ProApp. In one example, the QRC may be decoded after an unlock code is entered into the ProApp. by a first responder. The QRC may be associated with a file transfer as disclosed herein. In some embodiments, downloaded medical records are not automatically deleted to ensure access by first responders and other providers responding to the emergency. In some embodiments, client records are deleted after their initial use in non-emergency situations.

In some embodiments, a first responder may identify a current medical condition of the client when requesting access to medical records. In practice, the request for medical records may be automated, such that the first responder may initiate an application or module on the first responder device 526 in order to access medical records of the client. The application may be a customized emergency response application, and/or may comprise a provider application that includes an emergency procedure module. In some instances, the first responder may provide information related to the condition of the client and such information may be used to determine a subset of the client's medical records that can be provided to the first responder. The application may provide options and instructions that allow a first responder to operate the client device 502 in order to display the FREI 522 for capture using the first responder's first responder device 526.

In certain embodiments, the first responder's first responder device 526 may automatically generate and transmit a request for medical records upon capture of the FREI 522. The request may be handled by one or more medical records as discussed herein, but using a preauthorization of the client to access necessary or useful records.

In certain embodiments, first responders and other medical providers may connect with an embedded computing system to gain access to EHRs belonging to an individual when called to provide assistance to the individual. The embedded computing system may be deployed in a vehicle or a household appliance, for example. The embedded computing system may be configured to maintain information related to one or more registered users or identified users of a device that includes the embedded computing system. In one example, an on-board vehicle management system, entertainment system, navigation system or other controller or appliance may be adapted to identify an occupant of a vehicle such as an automobile in order to provide customized service to the occupant. Identification may be made by manual selection, RFID such as an RFID embedded in a key or vehicle access device, biometric information captured by a system of the vehicle (e.g. an iris or fingerprint scan).

In one example, an occupant of a vehicle may be identified through detection of wireless devices operated by the occupant, where the wireless devices may be a mobile phone, media player, a tablet computer, a laptop computer, and so on. The presence of multiple occupants of a vehicle may be known, although not all occupants may be identifiable by a device or appliance of the vehicle. The identity of an occupant may be used to customize the cabin environment of the vehicle by adjusting seat positions, configuring an audio device, defining frequently used routes for a GPS navigation system, etc. This identity may be associated with emergency response procedures configured and authorized by the identified occupant in advance. Other type of embedded computing systems in other devices and appliances may perform customizations based on identity of persons present in the vicinity of the devices or appliances.

Devices and appliances may be adapted to maintain information that can provide access to EHRs of a current occupant of a vehicle or user of an embedded device or appliance. In one example, FRIDs may be maintained or associated with each potential user of a device or known occupants of the vehicle. The device or appliance may also be adapted to maintain authorizations to be used in case of an emergency. Emergency information including FRIDs, FRID associations and/or emergency authorizations may be provided to devices and appliances using a mobile computing device of a record holder. For example, a record holder may operate an application installed on a mobile computing device to transfer and configure the emergency information on the device or appliance. The application may be an iBlueButton^{®} application, a configuration application provided by the vehicle manufacturer or supplier of a device or appliance. A device or appliance may visually or audibly greet a new device connected wirelessly or by wire and may invite a user of the new device to provide emergency response information. Typically, an owner of a vehicle, device or appliance may initiate a configuration process which offers an option to provide emergency information and to configure emergency response.

A first responder may automatically obtain authorization to access EHRs by interrogating a device or appliance and/or by responding to a communication initiated by the device or appliance. In one example, a first responder arriving at the scene of a traffic collision may obtain authorization to access EHRs of an injured occupant of a vehicle by providing an FRID to a device or appliance that maintains or has indicated it has access to emergency information of an occupant of the vehicle, and who may be the injured occupant. Upon validation of the FRID, the device or appliance may execute a proximity exchange such as one of the exchanges described in relation to FIGs. 3 and 4. Authorization to access the EHRs may be provided wirelessly and/or may involve transfer of information in a barcode displayed within the vehicle or on the device or appliance. In the example of a traffic collision, a vehicle may detect the collision and may provide emergency information through a remote diagnostics system such as systems operated by the OnStar^{™} Corporation. The information may then be forwarded for the use of first responders. Emergency information provided through vehicle monitoring systems may be encrypted such that only authorized third party responders may extract the encryption keys and identifiers necessary to access the EHRs of an injured occupant.

Emergency information maintained by a device or appliance may include some medical information that may be needed by a first responder even if access to EHRs is not sought. Such medical information may include information that identifies known medical conditions of the client that could render the client incapacitated, and/or that identify allergies suffered by the client, including drug allergies that could cause distress to the client if administered during an emergency situation, such as a traffic collision.

In some instances, automatically-initiated emergency authorizations to transfer EHRs may be rescinded by the owner of the EHRs. In one example, an occupant of a vehicle involved in a collision may be relatively uninjured and may respond to an alert of a device or appliance instructing the device or appliance that no transfer of EHRs should be performed. In another example, the uninjured occupant may block transfers of EHRs through an application (e.g. the iBlueButton^{®} application) installed on a mobile computing device.

A proximity exchange may be executed in response to a medical emergency, potential medical emergency or other event.

In a first example, the user device 502 may receive a user-generated alarm or an alert from a user. The user device 502 may be adapted or configured to present an icon image or text that enables a user to generate an alarm or alert. In one example, an icon or image (here an SOS button 508) may be presented on a login display 504 or in a display presented by an application configured to support or interact with EHR access. In this example, the user may select the SOS button 508 to signal the alarm or alert. The user device 502 may prompt the user for confirmation of the request for assistance. If the request is confirmed, or if no response is received, the user device 502 may initiate an emergency request according to a recognized or configured methodology.

In a second example, the user device 502 may receive a user-generated alarm or an alert when the user device 502 is configured to display an SOS button 516 on an idle screen 514 and/or when a medical-related application has control of the display. The user device 502 may prompt the user for confirmation of the request for assistance. If the request is confirmed, or if no response is received, the user device 502 may initiate an emergency request according to a recognized or configured methodology.

In a third example, the user device 502 may automatically generate an alarm or an alert. In one example, the user device 502 may be configured with a medical-monitoring and/or exercise application that interacts or monitors biometric sensors, and that can detect anomalies or irregularities. The user device 502 may prompt the user to determine if a request for assistance is needed or desired. If the request is confirmed, or if no response is received, the user device 502 may initiate an emergency request according to a recognized or configured methodology. In another example, the user device 502 may be configured to prompt the user on a periodic basis. The alert may be sent when medication or medical monitoring is to be performed. In some instances, the user may program the frequency of the prompts, and/or may set on-time prompts.

According to certain aspects, a request for assistance may be configured using information maintained by the user device 502 and/or generated by the user device 502 upon request. For example, the user device 502 may employ applications 510 that may serve as sources and/or repositories of information. In one example, the applications 510 may include a contacts manager that provides contact information related to the user, and/or to a medical provider associated with the user. In another example, the applications 510 may include an EHR application that may be configured to provide user medical records in accordance with certain aspects disclosed herein. In another example, the applications 510 may include a global positioning application that may be used to locate the user device 502 and provide geographic location to an emergency provider.

The user device 502 may send the request for help 512 after collecting contact, position and medical condition information from the applications 510 maintained by the user device 502. The user device 502 may then enter a mode of operation in which it is configured to respond to requests for information by a first responder or medical provider. For example, the user device 502 may authenticate a first responder or medical provider and provide updates of geographic position, and biometric readings while the first responder or medical provider is in transit. The user device 502 may be configured to activate a microphone, speaker and/or camera to permit interaction between the user and the first responder or medical provider. When the first responder or medical provider arrives at the location of the user, a proximity exchange 524 may be initiated to enable the first responder or medical provider to receive EHR information through the user device 520.

In one example, an EHR exchange may be secured by optically exchanging authentication information between the user device 502 and a first responder device 526. The devices 520 and 526 may be any suitable mobile processing and/or communication device such as a smartphone, tablet, a wearable computing device, a smart watch, a health or fitness tracker, eyewear, media player, appliance or other suitable device that is equipped with a camera or optical reader. The user device 502 may display a barcode (e.g. the FREI 522) in manner that enables the first responder device 526 to capture and decode the barcode. For example, a QR code may provide emergency authorization to permit any validated first responder device 526 to access EHR of the person seeking assistance. A validated first responder device 526 may, for example, carry or have access to encryption keys necessary to decode information in the QR code. An agent or application installed in the user device 502 provides access to EHR information related to the person seeking assistance. An agent or application installed in the first responder device 526 can receive the EHR information from the user device 520, and/or may access one or more systems as authorized through the operation of the QR code, the one or more systems including a practice management system, EHR systems 202, 204, 206 (see FIG. 2), and other systems (e.g. an aggregator 210).

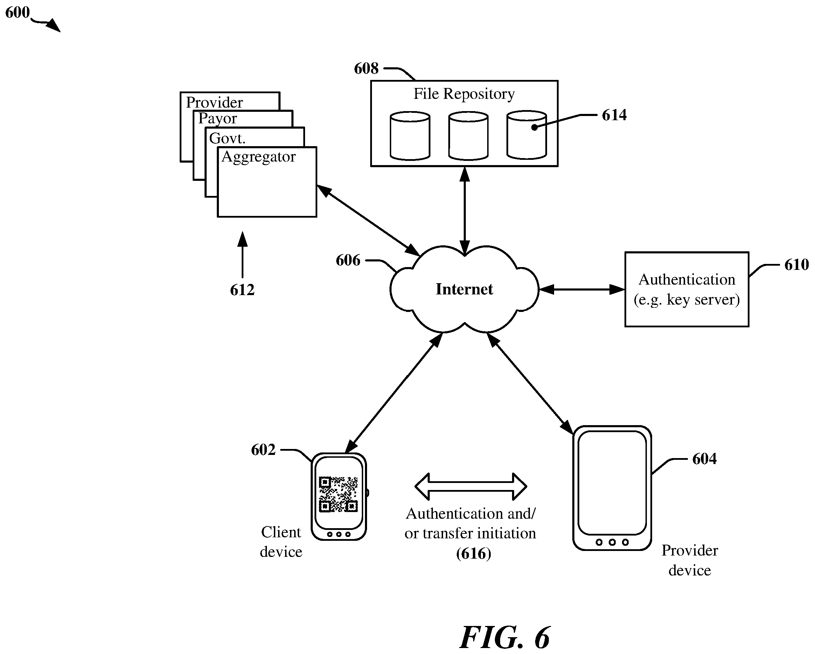

FIG. 6 illustrates a simplified example of a system that provides secured EHR exchange. Client device 602 may identify and/or prepare a set of EHR information for transfer to the provider device 604. For example, client device 602 may select EHR information from one or more sources to be transmitted to provider device 604. The EHR information may comprise records stored on client device 602. The EHR information may comprise records stored in one or more EHR systems and/or aggregators 612.

Client device 602 may then cause the selected EHR records to be stored in a file repository 608. File repository 608 may operate to provide a location for storage of a plurality of files and objects in a container 614 that can be uniquely identified and accessed through a network such as the Internet 605. The container 614 may be created for the duration of the EHR exchange and the container 614 may be destroyed when the contents have been forwarded to the provider device 604, or after a predetermined time. File repositories may be implemented using an Internet cloud service such as Dropbox^{™} or Amazon S3^{™}. The selected EHRs may be encrypted before being stored in the container 614.

The client device 602 and provider device 604 may exchange identifying and/or authenticating information in a proximity exchange 616 used to initiate the EHR exchange. In some embodiments, the client device 602 may provide information that enables access to the container 614 in an encoded optical image that is displayed by client device 602. The information in the encoded optical image may include one or more of an address of the file repository 608, a name of the container 614 that stores the EHRs selected by the client, an encryption key, and one or more usernames and passwords. The encoded optical image may be a QRC.

The provider may capture the encoded optical image and extract the location of the container 614 and encryption keys need to decrypt the contents of the container 614. Typically, in-person acknowledgement is available in a proximity exchange 616, and the provider device 604 does not typically provide an electronic message acknowledging capture of the optical image or even receipt of the EHRs to the client device 602. In at least some embodiments, electronic acknowledgement is made and such acknowledgements may be used for detailed logging of EHR exchanges by either the receiving or sending device. In some embodiments, the exchange of EHRs may be initiated by a provider and a patient may authorize transmission of EHRs to an address provided in an optical image displayed by provider device 604 and captured by client device 602.

In some embodiments, optical images may be transferred between devices 602 and 604 to enable direct communication of EHR records, to provide access to secured servers and/or to enable exchange of EHR information using encrypted Email or other communication systems. In some embodiments, optical images may be used to enable exchange of EHRs between parties connected by videoconference. For example, telemedicine may be employed to enable consultation between a physician specialist and a patient. Security for EHR exchange in such sessions may be augmented using encoded optical images captured from a videoconference display.

In certain embodiments, cryptographic keys may be exchanged by capturing an encoded image displayed on one or more of devices 602 or 604. An asymmetric key cryptographic process may be employed to improve security of the EHR exchange. Asymmetric key cryptography systems use two separate keys which are mathematically linked. The keys may be provided by an authentication service, which can generate public and private keys for the EHR exchange.

In certain embodiments, one or more logs may be configured to record the EHR exchange. When logging is required or preferred, components involved in the EHR exchange may provide affirmative acknowledgements of received information, including EHRs, content of EHR exchanges, authenticated user information, addresses of participants of EHR exchange, and/or date and time information corresponding to the EHR exchange. Logs may be maintained by the client device 602, provider device 604, EHR systems and/or aggregators 612, repository 608 and/or a container management system associated with repository 608, and authentication service providers 610. Logs may be consolidated, formatted, summarized and/or aggregated by one or more of the client device 602, provider device 604, EHR systems and/or aggregators 612, repository 608 and/or a container management system associated with repository 608, and authentication service providers 610. Typically, at least one of the client device 602, provider device 604, EHR systems and/or aggregators 612, repository 608 and/or a container management system associated with repository 608, and authentication service providers 610 maintains a log detailing one or more of a description of the EHRs stored in the container 614, or updated by the client or provider/recipient. Logs may also include information identifying the client, the recipient of the electronic healthcare records, and dates and times of transactions related to the electronic healthcare records stored in the container 614. Identification of members and providers may include member and/or provider numbers, biographic or demographic information as desired or permitted by regulatory authorities.

### Examples Of EHR And Other Information Exchanges

In certain embodiments, standardized health summaries can be made available to patients for easy download from government and private healthcare portals and to be shared with their healthcare providers. In some instances, immediate, proximate, secured exchange of health records and related health information is enabled between a patient and a physician or between two physicians. The exchange may be made in real time using mobile devices 302 and 308 (see FIG. 3). Certain embodiments of the invention enable secure and easy communication of EHR data from a patient device 302 to a physician device 308 over a local wireless network during a patient encounter with implicit or explicit patient consent. The exchange may take place in a physician's office, in an emergency room, an urgent care center, or at a hospital without a need to configure network servers and provider workstations with individual account names, addresses and security login parameters. A proximity exchange provides immediate access and secure exchange of individual health information at the time when the sender and the receiver of the information being exchanged can physically recognize each other and are reachable to each other over a network such as a wireless network.

In certain embodiments, a physician can exchange health information with a patient or with another physician using mobile devices 302, 308 and 314. The exchange can occur between two mobile phones, two tablet or other computers, or between a mobile phone and a tablet or other computer.

A patient device 302 may be adapted using an application or agent that securely stores and organizes personal health records and health information. The patient device 302 may be adapted using an application or agent that automatically accesses a patient portal account and can automatically login to retrieve current and updated patient health records. The patient device 302 may be further adapted to automatically download and combine health records from patient web portals using login and other identification and authentication maintained by the patient device 302.

In certain embodiments, the patient device 302 may be adapted to capture photographs of health documents and/or body parts using a camera in the mobile device 302. The patient device 302 may be adapted using an application or agent that accesses records created by other applications on the patient's mobile device. Proximity exchange may be used to transfer one or more health records and health information to a physician.

The patient device 302 may be adapted using an application or agent that directly receives health records, such as a visit summary, a referral note, test results, patient instructions, etc., from a physician using proximity exchange from the physician's mobile device 308.

The patient device 302 may be adapted using an application or agent that enables receipt of different types of records, including documents, photographs, audio and/or video recordings that may transferred by a physician using proximity exchange from the physician device 308 and the patient device 302 may be further configured to store and organize records exchanged to and from different physicians.

The physician device 308 may be adapted using an application or agent that can securely store and organize individual patient records and health information associated with several patients. The physician device 308 may be adapted using an application or agent that accesses records created by other applications, such as an electronic medical record (EMR) application, on the physician device 308.

The physician device 308 may be adapted using an application or agent that takes photographs of patient records and/or patient body parts using a camera of the physician device 308. The physician device 308 may be further adapted to create an audio recording, including follow-up care instructions, and to store such recordings as part of the patient's record on the physician device 308.

The physician device 308 may be adapted using an application or agent that directly receives health records from a patient, using proximity exchange from the patient's mobile device and that downloads health related information from a variety of provider, electronic medical record, health information exchange and other portals.

In some embodiments, either the patient or the doctor can initiate a proximity exchange. The initiator of the communication may push a button or otherwise activate a function of an agent or application of their respective mobile device 302 or 308. The initiator mobile device 302 or 308 may then broadcast over the wireless network an identification that may include a name that the other party can positively identify. The recipient may be notified that a request for proximity exchange has been received and may receive the name or names of the initiator. The recipient may choose between initiators detected within range of the recipient's mobile device 302 or 308 (e.g. a different physician and a different patient may be initiating an exchange in a nearby examining room). The proximity exchange may be authorized to commence when the recipient accepts the initiator.

In one example, Bluetooth and WiFi networks may be present. A mobile device may first attempt to advertise its desire to perform a proximity exchange using a WiFi Access Point (AP) if it is able to gain access to one within its wireless range. If the devices of both communication parties are able to access the same AP at the same time then the proximity exchange is performed through the AP, otherwise an attempt is made to connect them over Bluetooth. In some embodiments, Bluetooth connections are attempted first.

In certain embodiments, data is encrypted for transfer by proximity exchange. Encryption provides security that is not dependent upon on the security features of the underlying wireless network. Patient data such as health records and personal health information may be stored in encrypted form in mobile devices 302 and 308. In one example, encryption is performed using AES encryption algorithms with a secret encryption key that may be unique for the mobile device 302 or 308. The encryption keys may be generated during configuration and installation of the agent or application on the mobile device 302 or 308. Encryption keys may be based on a user password and a 64 byte random number. Encryption keys may be securely stored on the device in special secured hardware. This encryption protects both the confidentiality and the integrity of the data on the mobile devices 302 and 308.

Prior to transmission by proximity exchange, encrypted data may be first decrypted using the local cryptographic key of the sending device. The decrypted data may then be encrypted using a cryptographic key, which is known to both the sender and the receiver and which is created dynamically to exist only during the lifetime of the communication session. For example, the Diffie-Hellman algorithm may be used to create a communication session cryptographic key in such a way that only the two mobile devices 302 and 308 know the key. When encrypted data is received at the destination mobile device 308 or 302, it can be decrypted using the key associated with current proximity exchange and then re-encrypted using the local cryptographic key of the destination device before it is stored.

In certain embodiments, health records and related health information can be securely exchanged in real-time without the need for predefined network infrastructure. Proximity exchange may provide secure communication between two parties who can physically recognize each other and can communicate electronically with each other over a network.

In certain embodiments, personal identification and contact information can be exchanged between patient device 302 and physician device 308 as an option during proximity exchange. Personal identification information can include name, phone number, e-mail address, photograph, and such information may facilitate later contacts between the doctor and patient. In some embodiments, the contact information is exchanged automatically, without the requirement for each party to request it to be sent. Contact information may be automatically attached to records exchanged between the parties to enable easier filing and to enable accelerated retrieval on the respective mobile devices 302 and 308.

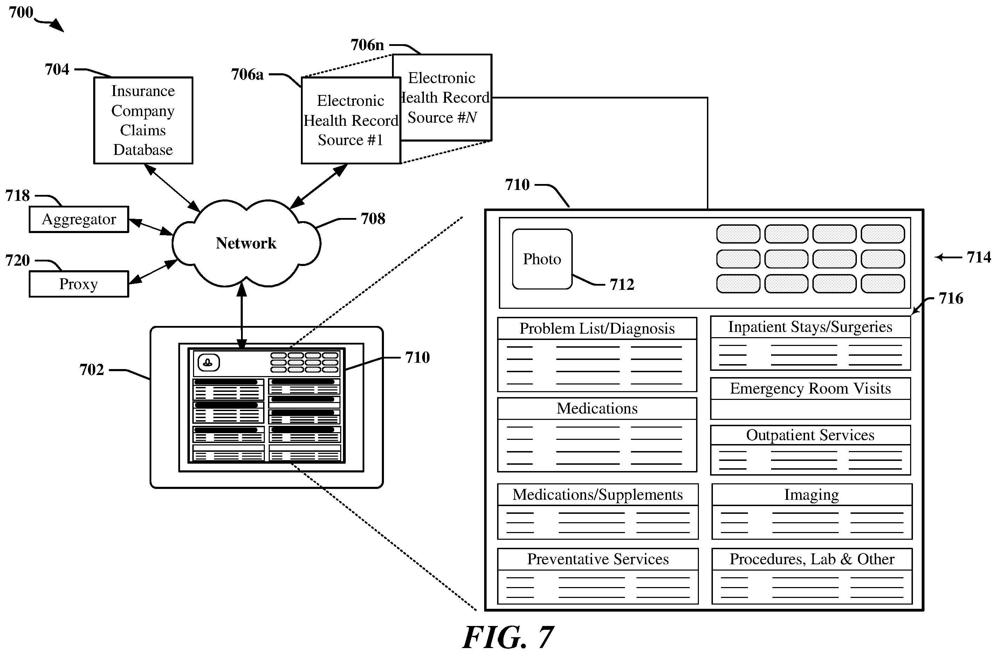

Record owners and providers may access the record owner EHR through a personalized portal provided on a mobile device or a conventional computing platform. Record owners may access their EHR information from a plurality of different sources and may provide one or more providers with partial or complete access to their EHR information. FIG. 7 illustrates a presentation of EHR information using a personalized portal according to certain aspects of the invention. The personalized portal may present a single display area that includes information from a plurality of sources including healthcare practitioners, insurance companies, an entity responsible for payment for services and other providers. EHR information may be combined remotely using a computer system or network server to access a plurality of EHR systems, before filtering and presenting the information to the record owner or provider. An aggregation server may reduce system complexity by providing identification, authentication, and qualification services related to the record owner and provider base as a centralized service, rather than requiring the plurality of EHR systems to maintain authentication information for the record owner and provider base. In some embodiments, a portal or agent may directly access and combine EHR information from the plurality of EHR systems.

Qualification services may filter results obtained from the plurality of EHR systems. Records received may be filtered based on certain predefined rules which may enforce government regulations. For example, certain records may not be accessible if access would cause healthcare information to be transferred between state or national jurisdictions. Records received may be filtered based on rules established by the record owner, a provider or the EHR system supplying the records. In one example, a record owner may determine a set of EHR records or a class of EHR records that should be withheld from one or more provider. The record owner may request that EHR records sent to a podiatrist should not include records related to psychiatric treatment, and vice versa.

An aggregator may format the information for display and/or may provide the information to an interface application that delivers a final format for display to the physician or other user. Interface application may be embodied in a portal or agent deployed on a record owner's computing device. Interface application may be provided as a plug-in on a network application at a provider location. Information provided by aggregator may be displayed in a web browser, a custom viewer application or in any suitable office automation application, such as a document reader or presentation tool. In certain embodiments, the display format may be specified and/or customized based on some combination of preferences and requirements of an end-user, a system administrator, a provider, payer and the record owner whose records are to be displayed. For example, the record owner may determine which fields are to be displayed and which data should be withheld. In another example, financial information is selected for display based on authorization levels set for the end-user.

In a certain embodiments, the record owner is a patient who receives, or expects to receive, healthcare services in a plurality of locations from multiple healthcare providers, such as his primary care provider (physician), a physician specialist and a pharmacy. The record owner may be insured by a private or public health insurance plan. Each provider may maintain separate and distinct electronic health records for the record owner. In some embodiments, record owner is permitted access to at least a portion of the records maintained by a provider on-line when such access is for the use of the record owner. For example, a record owner may access certain records from home to check on his insurance status, medical appointments, to view prescription refills, or communicate by e-mail with attending physicians.

Certain embodiments provide a record owner-controlled, practical, flexible, direct access to the record owner's health record that is continuously available. In some embodiments, the record owner may print and/or store a summary of online records on a removable storage device when it is necessary to present EHR records to one or more providers who are not users of the electronic delivery systems described herein. It will be appreciated however, that the printed or stored records are typically static and, if not updated in a timely manner, can become outdated by the time the records are presented at the point of care. Furthermore, the saved or printed record will typically not be available at all times, including during an emergency or at the time of a routine healthcare appointment, and may not be securely stored or carried; accordingly these stored or printed records can be subject to loss or tampering. Electronic access to EHR records may additionally resolve existing complex and ineffective patient consent management solutions, typically paper-based and single facility-based.

Consent may be provided by record owners as part of a request to deliver the record owner's EHR records. Certain embodiments provide direct access by healthcare providers to record owner records, whereby current record owner records are directly downloaded to the provider's system. The record owner may be required to provide authentication when requesting that a portion or all of the record owner's records are directly pushed to a provider system. In some embodiments, the record owner may also provide time-limited consent to permit a provider to request and access patient records directly from another service provider or from an aggregator. Consent may be provided directly by the record owner using a portal or agent, which may be implemented in a smart phone, a smart watch, a health or fitness tracker, eyewear, or other portable processing device.

### Examples Of Device Configuration And EHR Display

A portal or agent may be provided on a computing device. A portal may provide access to a record owner's EHR information through a browser or an application or agent that resides temporarily on the computing device. The portal may comprise an application that is downloaded and executed through a browser or loaded from a portable storage device, such as a USB drive. In one example, a USB drive may be used as a credential to identify and/or authenticate a user of the USB drive, through encryption keys, biometric information, etc., and may provide an application that enables the record owner to establish a portal on the computing device. The USB drive or another credential may be issued by his insurer, the government, or his primary healthcare provider system, etc., and may maintain record owner information such as a personal and unique identifier assigned to the record owner, a record locator address and login. The USB drive may also be configured to maintain a previously downloaded EHR document, typically in encrypted form.

The portal may comprise one or more downloadable applications and may deliver services performed by a network server. An agent may be installed or otherwise maintained by a computing device. The agent typically performs one or more functions that allow a record owner to access EHR information. The agent may identify a wireless device such as an RFID, a Bluetooth-enabled device, a WiFi connected device or another device that can be used to identify the user. The agent may be an application installed on a smart phone, tablet computer or notebook computer, whereby the record owner may use an identifier to gain access to EHR information. Identification may comprise a combination of user ID, password, challenge, biometric information such as a fingerprint, iris scan, facial scan effected by an on-board camera, and so on.

The agent or portal may be configured to perform a plurality of functions including record owner identification and authentication, access to EHR records, identification and authorization of EHR records to be pushed to a provider, aggregation of EHR records and direct push of EHR records from the record owner's personal portal to a provider's system.

In certain embodiments, a record owner may use a smart portable device that has a processor and storage. The record owner may connect a flash drive, smart card, a wirelessly connectable storage device, or the like to the computer. In one example, the record owner may present an NFC device, such as an RFID, a smart watch, a health or fitness tracker, eyewear, or smart phone that responds to or activates an NFC receiver on a provider computing workstation. The record owner may also exchange authentication information with a provider using an optical reader or camera capture barcodes displayed by user or provider, and/or to capture biometric information that automatically enables access to the EHR information. Additionally, a device-to-device communication protocol between the patient's device and a provider's portable device may be employed to automatically access and exchange electronic health records, or initiate such exchange, with the healthcare provider.

FIG. 7 is a diagram 700 illustrating an example of delivery of EHR information to a computing device 702. The computing device 702 may be operated by a healthcare provider, and may comprise a tablet computer, a desktop computer, a notebook computer, or any other suitable computing device. The computing device 702 may receive and display a summary form 710 based on a patient's EHRs. The summary form is typically generated "on-the-fly" and/or on-demand. The summary form 710 may be dynamically updated to reflect activities in progress, or to add delayed information received from one or more sources of information 704, 706a-706n. The summary form 710 may be generated using information retrieved from local sources or through a network 708 which may include a local area network and/or wide area network such as the Internet. The summary form 710 may be generated from information retrieved from one or more EHR sources 706a-706n, insurance claims databases 704, or other sources. The summary form 710 may be generated from information provided by an aggregator 718 which combines information retrieved from one or more EHR sources 706a-706n, insurance claims databases 704, or other sources. The summary form 710 may be generated by an application provided in the computing device 702 or a proxy device or server 720.

The summary form 710 may be navigable, whereby a user of the computing device 702 may select certain items 716 in the summary form 710 to obtain more detailed information. The summary form 710 may include controls 714 that permit a user of the computing device to initiate actions. In one example, the controls 714 may include a button or button icon that, when activated, causes the computing device 702 to retrieve additional information including contact information of the patient, providers or payers. In another example, the controls 714 may include a button or button icon that, when activated, causes the computing device 702 to view additional information related to a patient history, including a family history, allergies, immunizations and/or implanted devices. In another example, the controls 714 may include a button or button icon that, when activated, causes the computing device 702 to export or print information from the summary form 710 or other information provided in the downloaded EHRs.

The summary form 710 may be tailored to the requirements of the user, whether an EHR holder, an insurance provider, a government agency, a physician or other healthcare provider. The summary form may be formatted for ease of viewing on any suitable platform. The summary form may be presented in a single view, window and/or screen to allow a physician or patient to access desired information in one place, with a minimum of required navigation. This single screen display can be generated on the fly and can include clinical information (e.g. in CCD/CCR format), administrative information and financial information, such as insurance eligibility information and past utilization and encounter information. The healthcare provider can typically obtain immediate access to the type, amount and location of services received by a patient, as well as out of pocket expenses incurred.

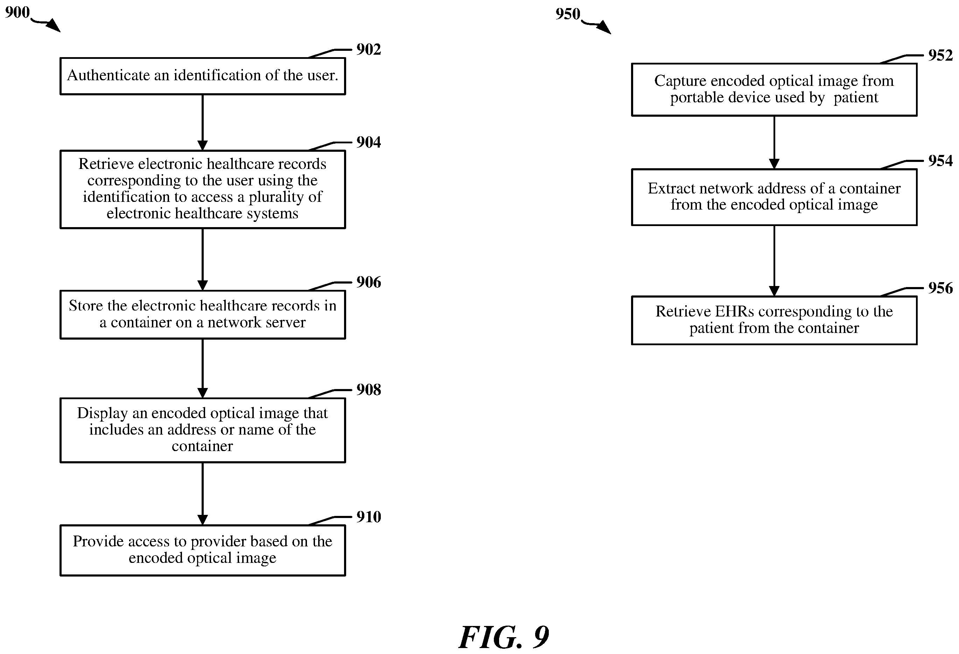

Certain processes according to certain aspects of the invention will now be described with reference to FIG. 9 and FIG. 2. For the purposes of the description, an example an embodiment of the invention used by military Veterans will be described, whereby a typical Veteran accesses healthcare at different Veterans Administration (VA) and non-VA provider sites and EHR information for the Veteran is maintained by government and non-government entities. In the example, an exchange can occur between points of care, whereby electronic health records, such as Blue Button records, can be automatically downloaded from various patient portals by a client device 214 or electronic credential 218, which has been adapted through the installation of an embedded application. Various patient portals may be accessed through client device 214, 216 and/or 218, the patient portals including "My HealtheVet" at the VA, TRICARE Online, and MyMedicare.gov, and other examples.

### Examples of Processing Circuits And Methods

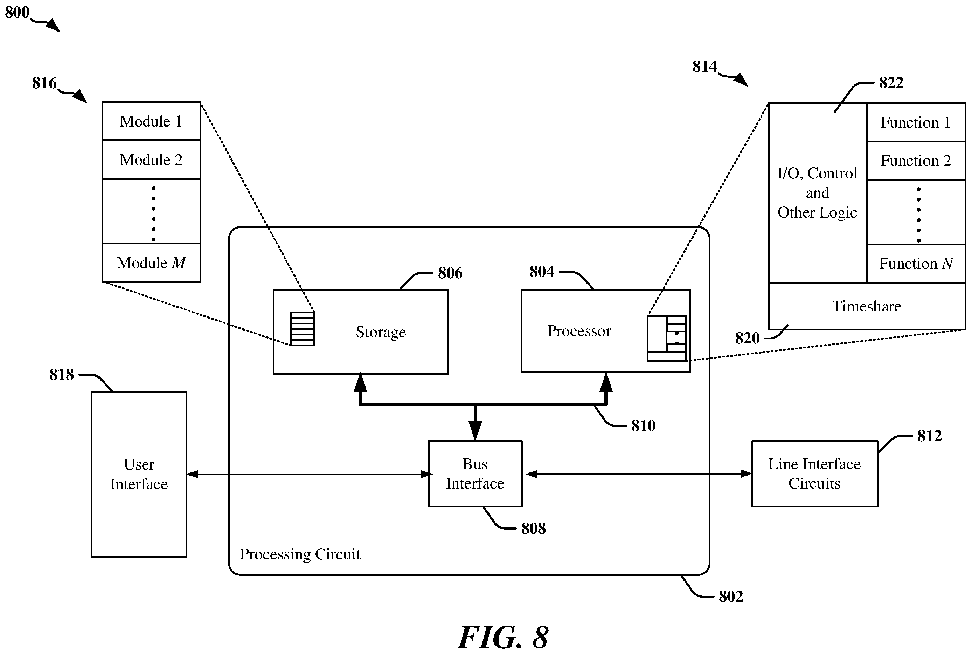

FIG. 8 is a conceptual diagram illustrating a simplified example of a hardware implementation for an apparatus 800 employing a processing circuit 802 that may be configured to perform one or more functions disclosed herein. In accordance with various aspects of the disclosure, an element, or any portion of an element, or any combination of elements as disclosed herein may be implemented using the processing circuit 802. The processing circuit 802 may include one or more processors 804 that are controlled by some combination of hardware and software modules. Examples of processors 804 include microprocessors, microcontrollers, digital signal processors (DSPs), ASICs, field programmable gate arrays (FPGAs), programmable logic devices (PLDs), state machines, sequencers, gated logic, discrete hardware circuits, and other suitable hardware configured to perform the various functionality described throughout this disclosure. The one or more processors 804 may include specialized processors that perform specific functions, and that may be configured, augmented or controlled by one of the software modules 816. The one or more processors 804 may be configured through a combination of software modules 816 loaded during initialization, and further configured by loading or unloading one or more software modules 816 during operation.

In the illustrated example, the processing circuit 802 may be implemented with a bus architecture, represented generally by the bus 810. The bus 810 may include any number of interconnecting buses and bridges depending on the specific application of the processing circuit 802 and the overall design constraints. The bus 810 links together various circuits including the one or more processors 804, and storage 806. Storage 806 may include memory devices and mass storage devices, and may be referred to herein as computer-readable media and/or processor-readable media. The bus 810 may also link various other circuits such as timing sources, timers, peripherals, voltage regulators, and power management circuits. A bus interface 808 may provide an interface between the bus 810 and one or more transceivers 812. A transceiver 812 may be provided for each networking technology supported by the processing circuit. In some instances, multiple networking technologies may share some or all of the circuitry or processing modules found in a transceiver 812. Each transceiver 812 provides a means for communicating with various other apparatus over a transmission medium. Depending upon the nature of the apparatus 800, a user interface 818 (e.g., keypad, display, speaker, microphone, joystick) may also be provided, and may be communicatively coupled to the bus 810 directly or through the bus interface 808.

A processor 804 may be responsible for managing the bus 810 and for general processing that may include the execution of software stored in a computer-readable medium that may include the storage 806. In this respect, the processing circuit 802, including the processor 804, may be used to implement any of the methods, functions and techniques disclosed herein. The storage 806 may be used for storing data that is manipulated by the processor 804 when executing software, and the software may be configured to implement any one of the methods disclosed herein.

One or more processors 804 in the processing circuit 802 may execute software. Software shall be construed broadly to mean instructions, instruction sets, code, code segments, program code, programs, subprograms, software modules, applications, software applications, software packages, routines, subroutines, objects, executables, threads of execution, procedures, functions, algorithms, etc., whether referred to as software, firmware, middleware, microcode, hardware description language, or otherwise. The software may reside in computer-readable form in the storage 806 or in an external computer-readable medium. The external computer-readable medium and/or storage 806 may include a non-transitory computer-readable medium. A non-transitory computer-readable medium includes, by way of example, a magnetic storage device (e.g., hard disk, floppy disk, magnetic strip), an optical disk (e.g., a compact disc (CD) or a digital versatile disc (DVD)), a smart card, a flash memory device (e.g., a "flash drive," a card, a stick, or a key drive), a random access memory (RAM), a read only memory (ROM), a programmable ROM (PROM), an erasable PROM (EPROM), an electrically erasable PROM (EEPROM), a register, a removable disk, and any other suitable medium for storing software and/or instructions that may be accessed and read by a computer. The computer-readable medium and/or storage 806 may also include, by way of example, a carrier wave, a transmission line, and any other suitable medium for transmitting software and/or instructions that may be accessed and read by a computer. Computer-readable medium and/or the storage 806 may reside in the processing circuit 802, in the processor 804, external to the processing circuit 802, or be distributed across multiple entities including the processing circuit 802. The computer-readable medium and/or storage 806 may be embodied in a computer program product. By way of example, a computer program product may include a computer-readable medium in packaging materials. Those skilled in the art will recognize how best to implement the described functionality presented throughout this disclosure depending on the particular application and the overall design constraints imposed on the overall system.

The storage 806 may maintain software maintained and/or organized in loadable code segments, modules, applications, programs, etc., which may be referred to herein as software modules 816. Each of the software modules 816 may include instructions and data that, when installed or loaded on the processing circuit 802 and executed by the one or more processors 804, contribute to a run-time image 814 that controls the operation of the one or more processors 804. When executed, certain instructions may cause the processing circuit 802 to perform functions in accordance with certain methods, algorithms and processes described herein.

Some of the software modules 816 may be loaded during initialization of the processing circuit 802, and these software modules 816 may configure the processing circuit 802 to enable performance of the various functions disclosed herein. For example, some software modules 816 may configure internal devices and/or logic circuits 822 of the processor 804, and may manage access to external devices such as the transceiver 812, the bus interface 808, the user interface 818, timers, mathematical coprocessors, and so on. The software modules 816 may include a control program and/or an operating system that interacts with interrupt handlers and device drivers, and that controls access to various resources provided by the processing circuit 802. The resources may include memory, processing time, access to the transceiver 812, the user interface 818, and so on.

One or more processors 804 of the processing circuit 802 may be multifunctional, whereby some of the software modules 816 are loaded and configured to perform different functions or different instances of the same function. The one or more processors 804 may additionally be adapted to manage background tasks initiated in response to inputs from the user interface 818, the transceiver 812, and device drivers, for example. To support the performance of multiple functions, the one or more processors 804 may be configured to provide a multitasking environment, whereby each of a plurality of functions is implemented as a set of tasks serviced by the one or more processors 804 as needed or desired. In one example, the multitasking environment may be implemented using a timesharing program 820 that passes control of a processor 804 between different tasks, whereby each task returns control of the one or more processors 804 to the timesharing program 820 upon completion of any outstanding operations and/or in response to an input such as an interrupt. When a task has control of the one or more processors 804, the processing circuit is effectively specialized for the purposes addressed by the function associated with the controlling task. The timesharing program 820 may include an operating system, a main loop that transfers control on a round-robin basis, a function that allocates control of the one or more processors 804 in accordance with a prioritization of the functions, and/or an interrupt driven main loop that responds to external events by providing control of the one or more processors 804 to a handling function.

FIG. 9 includes a flowchart 900 that describes a method employing a records access system that may provide access to a provider to client records. In one example, the records comprise EHRs, the client may be a patient and the provider may be a healthcare provider.

At step 902, the client device 214 may authenticate an identification of the user.

At step 904, the client device 214 may retrieve electronic healthcare records corresponding to the user by using the identification to access a plurality of electronic healthcare systems.

At step 906, the client device 214 may store the EHRs in a container 614 on a network server.

At step 908, the client device 214 may display an encoded optical image that includes an address or name of the container 614. The optical image may comprise a QRC, and/or another form of matrix code or barcode. The optical image may enable an intended recipient of the EHRs to retrieve the EHRs from the container 614. The EHRs stored in the container 614 may be encrypted, and the encoded optical image may include one or more keys necessary to decrypt the EHRs retrieved from the container 614.

The optical image may be captured by a computing system used by the provider or the patient. The computing system may comprise a computer or mobile computing device that includes, or is coupled to, a camera or other optical sensor.

At step 910, the provider or the patient may access the EHRs in the container 614 using information extracted from the optical image.

The client device 214 and/or the computing system used by the provider may comprise a wireless telephone, a smart phone, a wearable computing device, a smart watch, a health or fitness tracker, eyewear, and/or a tablet computer and wherein the portable computing device is configured to retrieve the information from the plurality of electronic healthcare records systems using a cellular wireless telephone network.

In some embodiments, the intended recipient of the electronic healthcare records receives the encoded optical image through a videoconference connection.

In some embodiments, the EHRs stored in the container 614 may be deleted after a predetermined time or may be deleted after a first retrieval from the container 614.

In some embodiments, at least one of the portable computing device, the network server and a computing device associated with the recipient maintains a log detailing one or more of a description of the electronic healthcare records stored in the container 614, the identity of the user, information identifying an actual recipient of the electronic healthcare records, and dates and times of transactions related to the electronic healthcare records stored in the container 614.

Veteran patient may present an ID card 218 that comprises a USB flash drive. The ID card may enable automatic communication/exchange of online health records with a provider EHR system 202. At step 904, software embedded in the Veteran's card 218 is automatically loaded and executed upon insertion and/or detection by an Internet-ready computing device 216. Typically, no software or system integration is requires and the software may directly launch a login screen for entry of the Veteran's single chosen password in order to grant the provider consent of the patient to proceed.

At step 906, the device embedded software may then auto launch and automatically login into one or more of the Veteran's selected EHR enabled patient portals. The computing device 216 may then download and combine EHR records, automatically and as directed by the device embedded software. The device embedded software may additionally reformat the downloaded EHR information into a clinically prioritized format in a single view (see FIG. 7). This single view may also include a reply prompt window for the provider to send, at step 910, a follow up note, with or without attachments, to the Veteran's primary care or referring physician. The follow up note may be transmitted by secure Email, Fax and/or secure messaging.

As shown in Fig. 2, a client device 214, 216 may comprise a smart phone, a wearable computing device, a smart watch, a health or fitness tracker, eyewear, or tablet computer on which an application or agent has been installed or embedded. The application or agent may adapt the client device 214, 216 to maintain at least a summary report of EHR records on the device. The application or agent may also adapt the client device 214, 216 to automatically access one or more EHR portals and store the EHR records in a container, or receive EHR records via the Direct protocol. In some embodiments, records can be pushed to a device operated by a provider (e.g., the physician device 212) upon consent and authentication of the Veteran. The records may be pushed to a physician device 212 using, for example, a service discovery protocol. An application or agent on the physician device 212 may signal its presence, which enables the Veteran to execute a transfer of records by commanding device 214 to directly push selected records to the physician device 212. The provider may be prompted to choose whether or not to accept the Veteran's records before or after transmission of the records by the client device 214, 216.

The physician may optionally provide updates records to client device 214, 216 or 218 which may then be relayed to the EHR systems 202, 204, or 206 through one or more portals. Typically, the provider reviews the received records and is provided a reply prompt to send information to the client device 214, 216. For example, the information sent by the physician may include a follow up note to the Veteran's primary care or referring physician. Optionally information such as a follow-up note may be transmitted by secure Email, Fax and/or secure messaging.

FIG. 9 also includes a flowchart 950 that describes a method employing a records access system that may provide access to a provider to patient records. In one example, the records comprise EHRs, the client may be a patient and the provider may be a healthcare provider.

At step 952, a computing device associated with a provider of healthcare services may capture an encoded optical image from a portable computing device presented by a patient. The encoded optical image may comprise a QRC or other barcode.

At step 954, the computing device associated with a provider of healthcare services may extract an address of a container 614 from the encoded optical image. The container 614 may be located on a network server. EHRs may be stored in the container 614. The EHRs stored in the container 614 may be encrypted. The encoded optical image may include one or more keys necessary to decrypt the electronic healthcare records stored in the container 614.

At step 956, the computing device associated with a provider of healthcare services may retrieve electronic healthcare records corresponding to the patient from the container 614. The EHRs stored in the container 614 may be deleted after a predetermined time, and/or after a first retrieval.

The computing device associated with the provider may comprise one or more of a wireless telephone, a smart phone, a wearable computing device, a smart watch, a health or fitness tracker, eyewear, and a tablet computer. The computing device associated with the provider may be proximately located with the portable computing device. In some embodiments the computing device associated with the provider may be remote with respect to the portable computing device, and the encoded optical image may be received through a videoconference connection.

In some embodiments one or more components of the system may maintain a log of transactions associated with the user and/or the EHRs. At least one of the portable computing device, the network server and the computing device associated with the provider may maintains a log that details one or more of a description of the electronic healthcare records provided in the container 614, the identity of the patient, information identifying the provider times of transactions related to the electronic healthcare records stored in the container 614.

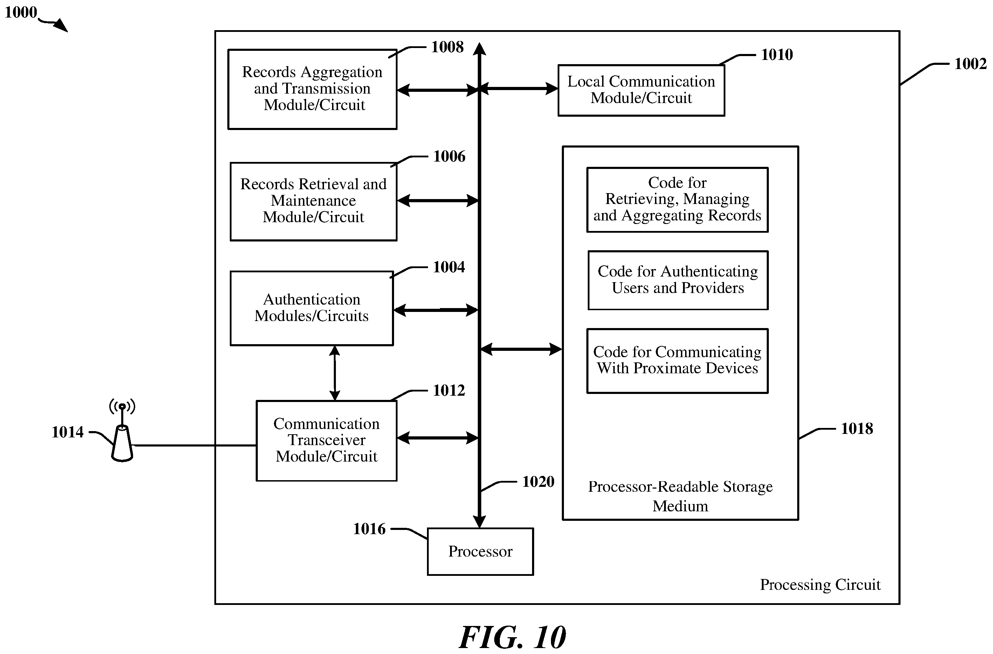

FIG. 10 is a block diagram illustrating the functionality of an apparatus 1000 employing a processing circuit 1102 as used in a provider location for accessing medical records. The apparatus 1000 may be a portable or non-portable computing device, having a processor 1016 and non-transitory storage 1018 in which an agent or software may be installed that includes one or more modules 1004, 1006, 1008, 1010 and 1012.

The apparatus 1000 may include an authentication module 1004 configured to identify and/or authenticate the user associated with the apparatus 1000. Module 1004 may identify the user using a biometric measurement, a password, user identifier, RFID device and/or a challenge.

The apparatus 1000 may include a records retrieval module 1006 that is configured to automatically retrieve information corresponding to the one user from at least one electronic healthcare records system using the identification to access the at least one electronic healthcare records system. The apparatus 1000 may retrieve the information from the at least one electronic healthcare records system using a cellular wireless telephone network.

The apparatus 1000 may include a records delivery module 1008 configured to electronically deliver a portion of the information to a healthcare provider. The apparatus may deliver the information using transceiver 1012 and antenna 1014, which may be configured to support Bluetooth communications and/or communications through a wireless network, such as a WLAN or cellular network. Accordingly, the apparatus 1000 may comprise a wireless telephone, a smart phone, a wearable computing device, a smart watch, a health or fitness tracker, eyewear, and/or a tablet computer. A portion of the information may be delivered to a different computing device operated by the healthcare provider. A portion of the information is delivered using a server communicatively coupled to the portable computing devices associated with the one user and operated by the healthcare provider. A portion of the information may be encrypted.

The apparatus 1000 may include a local connection module 1010 that establishes a data and/or audio-visual link with a provider. The apparatus 1000 may establish a connection using transceiver 1012 and antenna 1014, which may be configured to support Bluetooth communications and/or communications through a wireless network, such as a WLAN or cellular network. Accordingly, the apparatus 1000 may comprise a wireless telephone, a smart phone, a wearable computing device, a smart watch, a health or fitness tracker, eyewear, and/or a tablet computer. Module 1010 may perform other functions, including automatically providing consent to allow providers to download records or the user.

The apparatus 1000 may include an aggregation module 1008 that combines the retrieved information with other information retrieved from the at least one electronic healthcare records system to obtain combined information. The other information may comprise electronic health records of the user that are maintained by the apparatus 1000. Electronic health records maintained by the apparatus may be encrypted using encryption keys uniquely associated with the one user.

One or more of modules 1004, 1006, 1008, 1010 and 1012 may combine to perform a method comprising the steps of receiving from a first portable computing device, information identifying a user of the first portable computing device and a request for selected healthcare records corresponding to the user and an identity of a healthcare provider, causing the first portable computing device to authenticate identity of the user, wherein the authentication of the identity of the user serves as a consent of the user to release the selected healthcare records, and upon receiving information confirming the authentication of the identity of the user, transferring the selected healthcare records to a second computing device operated by the healthcare provider. In some embodiments the portable computing device maintains encrypted information that identifies the user.

The method may further comprise updating at least a portion of the selected healthcare records using information received from the healthcare provider. The method may further comprise healthcare records other than the selected healthcare records using information received from the healthcare provider. The method may further comprise creating new healthcare records using information received from the healthcare provider.

In some embodiments, the selected healthcare records comprise records from a plurality of sources, including at least one provider source and a payer source. In some embodiments, transferring the selected healthcare records includes receiving an acceptance from the healthcare provider. In some embodiments, the user and the healthcare provider are located in close proximity and wherein the transferring the selected healthcare records is contingent on a direct visual identification made by one or more of the user and the healthcare provider. In some embodiments, the user and the healthcare provider are located in different rooms and wherein the transferring the selected healthcare records is contingent on a virtual visual identification made by one or more of the user and the healthcare provider.

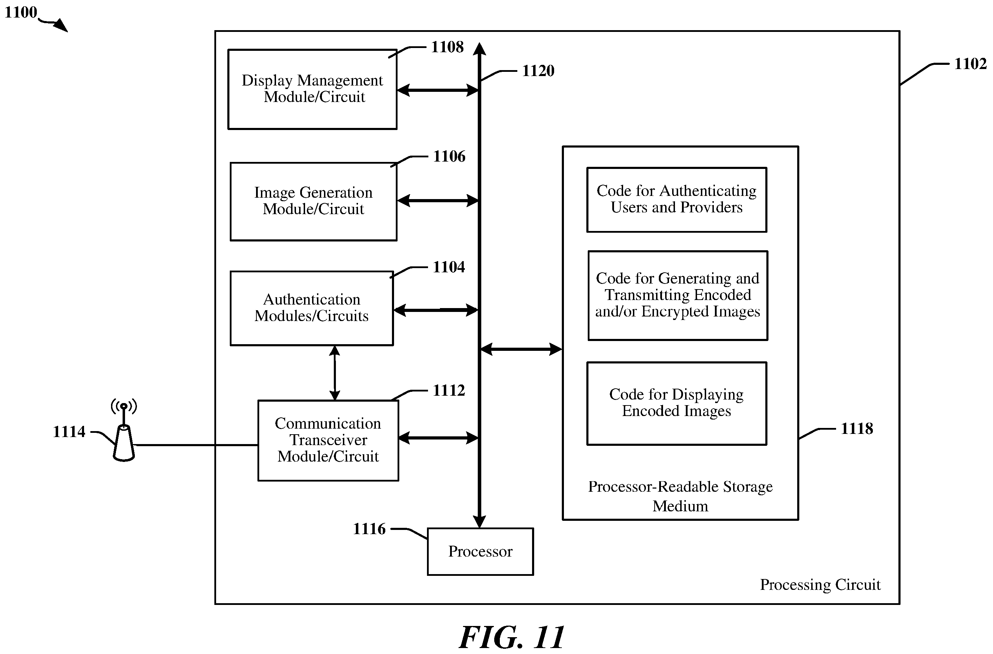

FIG. 11 is a diagram illustrating a simplified example of a hardware implementation for an apparatus 1100 employing a processing circuit 1102. The processing circuit 1102 typically has a processor 1116 that may include one or more of a microprocessor, microcontroller, digital signal processor, a sequencer or a state machine. The processing circuit 1102 may be implemented with a bus architecture, represented generally by the bus 1120. The bus 1120 may include any number of interconnecting buses and bridges depending on the specific application of the processing circuit 1102 and the overall design constraints. The bus 1120 may interconnect various circuits including processors and/or hardware modules, represented by the processor 1116, the modules or circuits 1104, 1106 and 1108, a transceiver 1112 configurable to communicate wirelessly through an antenna 1114 and the computer-readable storage medium 1118. The bus 1120 may also link various other circuits such as timing sources, peripherals, voltage regulators, and power management circuits, which are well known in the art, and therefore, will not be described any further.

The processor 1116 may be responsible for general processing, including the execution of software stored on the computer-readable storage medium 1118. The software, when executed by the processor 1116, may cause the processing circuit 1102 to perform certain of the functions described *supra* for any particular apparatus. The computer-readable storage medium 1118 may also be used for storing data that is manipulated by the processor 1116 when executing software, including data encoded in images and symbols transmitted wirelessly. The processing circuit 1102 further includes at least one of the modules 1104, 1106 and 1108. The modules 1104, 1106 and 1108 may be software modules running in the processor 1116, resident/stored in the computer-readable storage medium 1118, one or more hardware modules coupled to the processor 1116, or some combination thereof. The modules 1104, 1106 and 1108 may include microcontroller instructions, state machine configuration parameters, or some combination thereof.

In one configuration, the apparatus 1100 includes a module and/or circuit 1104 that is configured to authenticate an identification of a user of a mobile device, a module and/or circuit 1108 or 1112 that is configured to communicate an electronic authorization from the mobile device to a provider device using a first communication method. The electronic authorization may enable the provider device to have access to electronic healthcare records of the user. The access to the electronic healthcare records of the user may be provided through a second communication method that is different from the first communication method. In one example, the first communication method is initiated by the mobile device after the user of the mobile device has been authenticated, and comprises transferring an image between the mobile device and the provider device. The image may be generated by the module and/or circuit 1106 that may be configured to encode information identifying the user of the mobile device, and an address of the electronic healthcare records of the user. A module and/or circuit 1108 may be configured to display the image using a display. The image may be captured from the display by a camera of the provider device. The display may be provided as an internal or integral component of the apparatus 1100, or the processing circuit 1102. The display may comprise an external display system, such as a videoconferencing display that is controlled or operated through the processing circuit 1102.

In one example, the apparatus 1100 may comprise a mobile device, which may be configured to authenticate an identification of a user of a mobile device or other apparatus 1100 and communicate communicating an electronic authorization from the mobile device to a provider device using a first communication channel. The electronic authorization may enable the provider device to access EHRs of the user. Access to the electronic healthcare records of the user may be provided through a second communication channel that is different from the first communication channel. The first communication channel may be used by the mobile device to transfer an image between the mobile device and the provider device after the user of the mobile device has been authenticated. The image may be displayed by the mobile device for capture by a camera of the provider device. The image may include encoded information identifying the user of the mobile device. The image may include an address of the electronic healthcare records of the user. The image may include cryptographic keys.

The image may be displayed by the mobile device for capture by a camera of the provider device. The provider device may be configured to use the cryptographic keys to access the electronic healthcare records of the user. The image may include a QRC or a barcode.

The first communication channel may include a video link through a network connecting the mobile device and the provider device. The first communication channel may be a network controlled by a Near Field Communications protocol, a Bluetooth protocol or a Zigbee protocol. The second communication channel may include a wide area network that is configured to provide access to a container 614 on a network server. The EHRs of the user may be encrypted. The EHRs of the user may be deposited in the container 614. The EHRs of the user deposited in the container 614 may be deleted after a predetermined time. The EHRs of the user deposited in the container 614 may be deleted after a first retrieval of the electronic healthcare records of the user from the container 614.

At least one of the mobile device, the provider device and the network server may maintain a log related to transactions involving the container 614. The log may record a description of the EHRs deposited in the container 614. The log may record the identity of the user of the mobile device. The log may record an identity of the provider device when the provider device accesses the container 614.

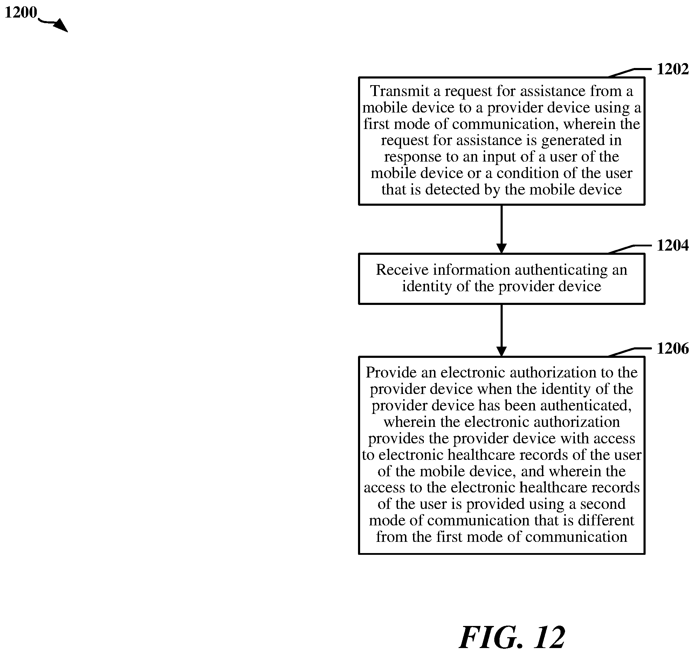

FIG. 12 includes a flowchart 1200 that describes a method for controlling access to electronic medical records. The method may be performed at a mobile computing device.

At step 1202, the mobile computing device may transmit a request for assistance to a provider device using a first mode of communication. The request for assistance may be generated in response to an input of a user of the mobile device or a condition of the user that is detected by the mobile computing device.

At step 1204 the mobile computing device may receive information authenticating an identity of the provider device.

At step 1206 the mobile computing device may provide an electronic authorization to the provider device when the identity of the provider device has been authenticated. The electronic authorization may provide the provider device with access to electronic healthcare records of the user of the mobile device. The access to the electronic healthcare records of the user may be provided using a second mode of communication that is different from the first mode of communication.

In one example, the first mode of communication is used by the mobile computing device to transfer an image between the mobile computing device and the provider device. The image may be displayed by the mobile device for capture by a camera of the provider device. The image may include encoded information identifying the user of the mobile device. The image may include an address of the electronic healthcare records of the user. The image may comprise a QRC or a barcode that includes cryptographic keys. The image may be displayed by the mobile computing device for capture by a camera of the provider device. The provider device may be configured to use the cryptographic keys to access the electronic healthcare records of the user.

In some examples, the mobile computing device may receive information authenticating the identity of the provider device, and may transmit the electronic authorization to a network repository of the electronic healthcare records of the user. The electronic authorization may serve as a consent to transmit the electronic healthcare records of the user from the network repository to the provider device.

In some instances, the mobile computing device may receive information authenticating the identity of the provider device and may retrieve the electronic healthcare records of the user from the network repository. The mobile computing device may transmit the electronic healthcare records of the user from the mobile device to the provider device.

In some examples, one or more code sets are maintained by the mobile computing device. The mobile computing device may enable a user to parse, decode, aggregate, classify or organize the electronic healthcare records of the user by category. The code sets may include international or national standardized health data code lists for medications, diagnoses, laboratory tests, procedures, immunizations, or individual medical professions. The electronic healthcare records may be displayed, updated or manipulated in a spoken language of a healthcare professional making use of the electronic healthcare records. In one example, the spoken language of the healthcare professional is determined automatically by a GPS-derived location or using another location service.

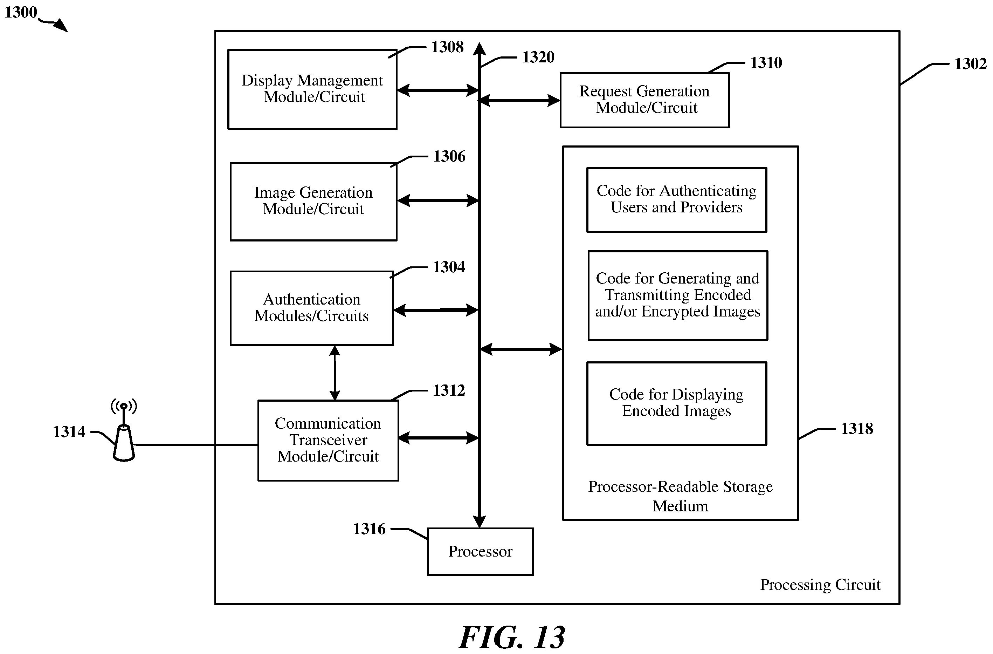

FIG. 13 is a diagram illustrating a simplified example of a hardware implementation for an apparatus 1300 employing a processing circuit 1302. The processing circuit 1302 typically has a processor 1316 that may include one or more of a microprocessor, microcontroller, digital signal processor, a sequencer or a state machine. The processing circuit 1302 may be implemented with a bus architecture, represented generally by the bus 1320. The bus 1320 may include any number of interconnecting buses and bridges depending on the specific application of the processing circuit 1302 and the overall design constraints. The bus 1320 may interconnect various circuits including processors and/or hardware modules, represented by the processor 1316, the modules or circuits 1304, 1306, 1308 and 1310, a transceiver 1312 configurable to communicate wirelessly an antenna 1314 and the computer-readable storage medium 1318. The bus 1320 may also link various other circuits such as timing sources, peripherals, voltage regulators, and power management circuits, which are well known in the art, and therefore, will not be described any further.

The processor 1316 may be responsible for general processing, including the execution of software stored on the computer-readable storage medium 1318. The software, when executed by the processor 1316, may cause the processing circuit 1302 to perform certain of the functions described *supra* for any particular apparatus. The computer-readable storage medium 1318 may also be used for storing data that is manipulated by the processor 1316 when executing software, including data encoded in images and symbols transmitted wirelessly. The processing circuit 1302 further includes at least one of the modules 1304, 1306, 1308 and 1310. The modules 1304, 1306, 1308 and 1310 may be software modules running in the processor 1316, resident/stored in the computer-readable storage medium 1318, one or more hardware modules coupled to the processor 1316, or some combination thereof. The modules 1304, 1306, 1308 and 1310 may include microcontroller instructions, state machine configuration parameters, or some combination thereof.

In one configuration, the apparatus 1300 includes modules and/or circuits 1306, 1308, 1310, 1312 that are configured to transmit a request for assistance to a provider device, modules and/or circuits 1304, 1312 configured to receive information authenticating an identity of the provider device, and modules and/or circuits 1304, 1312 configured to provide an electronic authorization to the provider device when the identity of the provider device has been authenticated.

It is understood that the specific order or hierarchy of steps in the processes disclosed is an illustration of exemplary approaches. Based upon design preferences, it is understood that the specific order or hierarchy of steps in the processes may be rearranged. The accompanying method claims present elements of the various steps in a sample order, and are not meant to be limited to the specific order or hierarchy presented.

### Additional Descriptions of Certain Aspects Of The Invention

The image may be displayed by the mobile device for capture by a camera of the provider device. The provider device may be configured to use the cryptographic keys to access the electronic healthcare records of the user. The image may include a QRC or a barcode.

Certain aspects relate to systems and methods that enable immediate access to actionable personal health information. The personal health information may be accessed anywhere at any time. Health care quality, safety and cost-effectiveness rely on the availability of up to date and actionable personal health information at any point of care. The present invention provides a combination of innovative functionalities embedded into a mobile application running on a patient's mobile device to access, transform, aggregate and annotate personal health information so that the medical information necessary for a patient to communicate or exchange with a healthcare professional is available at all times, including offline when Internet access is not available, and in the spoken language of that health care professional to render the right care at the right time is available to any healthcare professional.

In some examples, a system may include a computing mobile device held by an individual patient running a mobile application which provide functionalities to display the individual's medical history are available offline (without Internet connection). The functionalities may include local (i.e., on a user mobile device) parsing, decoding, aggregation, classification and organization by category (such as medications, diagnoses, laboratory tests, imaging services, provider names and contact information, etc.) of the individual personal health information extracted from either health insurance claims or electronic medical records.

Certain code sets necessary for the individual data decoding are may be included or provided to the application so that the decoding and other above application functionalities are occurring in real time, and do not require an Internet access (as opposed to server based processing), so that the transformed data are available at all times. The code sets may include the various international or national standardized health data code lists for medications, diagnoses, laboratory tests, procedures, immunizations, individual medical professions, and so on. Code sets specific to geographic regions or countries may be provided or maintained, and the application allows for the display of the individual data in the language of the user's choice or the health care professional accessing that data.

According to certain aspects, individual data can be displayed in the spoken language of the healthcare professional making use of that data can be automated based on the GPS location of the individual app user or the GPS location of the health care professional accessing that data via mobile to mobile communication in various means (QR code scanning, blue tooth, Bonjour or other "Bump" technology, etc.). When translated, individual medications and immunization data are matched to the corresponding specific data where the information is being reviewed (e.g., the American medication name initially entered by an American app user, or extracted from an American medical record system will display the corresponding generic name, or brand name or both for the healthcare professional receiving or viewing that data in the country visited by the user).

According to certain aspects, the displayed individual or aggregated records are actionable by a user who can edit each data element with personal annotations; selecting his own spoke language the use can add language and region specific health information. The displayed record data elements may be linked to various code sets so that they are searchable by the user with the link of each data element to online health information databases (e.g., Medline Plus in the U.S.A, NHS Choices in England, or Ameli.fr in France).

The previous description is provided to enable any person skilled in the art to practice the various aspects described herein. Various modifications to these aspects will be readily apparent to those skilled in the art, and the generic principles defined herein may be applied to other aspects. Thus, the claims are not intended to be limited to the aspects shown herein, but is to be accorded the full scope consistent with the language claims, wherein reference to an element in the singular is not intended to mean "one and only one" unless specifically so stated, but rather "one or more." Unless specifically stated otherwise, the term "some" refers to one or more.

## Claims

1. A method for controlling access to electronic healthcare records, the method comprising:
transmitting a request for assistance from a mobile device to a provider device using a first mode of communication, wherein the request for assistance is generated in response to a medical condition of a user that is detected by the mobile device;
in response to the request for assistance, receiving by the mobile device information authenticating an identity of the provider device; and
providing by the mobile device an electronic authorization to the provider device when the identity of the provider device has been authenticated, the electronic authorization proving consent of the user for the provider device to access electronic healthcare records of the user from a plurality of Electronic Health Record, EHR, systems maintained by at least two different providers;
wherein the electronic authorization provides the provider device with access to electronic healthcare records of the user of the mobile device, and
wherein the access to the electronic healthcare records of the user is provided using a second mode of communication that is different from the first mode of communication, and further wherein the access to the electronic healthcare records of the user is provided from an aggregator, wherein, the electronic authorization enables the aggregator to deliver a combined individual electronic healthcare record of the user sourced from the plurality of EHR systems to the provider device.

2. The method of claim 1, wherein the first mode of communication is used by the mobile device to transfer an image between the mobile device and the provider device.

3. The method of claim 2, wherein the image is displayed by the mobile device for capture by a camera of the provider device, and wherein the image includes encoded information identifying the user of the mobile device, and address information used to access the electronic healthcare records of the user.

4. The method of claim 2, wherein the image comprises a quick response code or a barcode that includes cryptographic keys and is displayed by the mobile device for capture by a camera of the provider device, and wherein the provider device is configured to use the cryptographic keys to access the electronic healthcare records of the user.

5. The method of claim 1, further comprising:
transmitting the electronic authorization to a network repository of certain electronic healthcare records of the user maintained by the network repository,
wherein the electronic authorization serves as a consent to transmit the electronic healthcare records of the user from the network repository to the provider device.

6. The method of claim 5, further comprising:
retrieving the certain electronic healthcare records of the user from the network repository; and transmitting the electronic healthcare records of the user to the provider device.

7. The method of claim 1, wherein one or more code sets that include an international or national standardized health data code list are maintained by the mobile device, and further comprising:
enabling a user to parse, decode, aggregate, classify or organize the electronic healthcare records of the user by category based on the one or more code sets.

8. The method of claim 7, wherein the code sets include international or national standardized health data code lists for medications, diagnoses, laboratory tests, procedures, immunizations, or individual medical professions.

9. The method of claim 7, wherein electronic healthcare records are displayed, updated or manipulated in a language spoken by a healthcare professional making use of the electronic healthcare records.

10. The method of claim 9, wherein the language spoken by the healthcare professional is determined automatically by GPS location or other location service.

11. A mobile computing device (602) configured for wireless communications and comprising:
at least one processing circuit, wherein the at least one processing circuit is configured to: cause a request for assistance to be transmitted from the mobile computing device (602) to a provider device (604) using a first mode of communication, wherein the request for assistance is generated in response to a medical condition of a user that is detected by the mobile computing device (602);
receive, in response to the request for assistance, information authenticating an identity of the provider device (604); and
provide an electronic authorization to the provider device (604) when the identity of the provider device (604) has been authenticated, the electronic authorization proving consent of the user for the provider device (604) to access electronic healthcare records of the user from a plurality of Electronic Health Record, EHR, systems maintained by at least two different providers;
wherein the electronic authorization provides the provider device (604) with access to electronic healthcare records of the user of the mobile computing device (602), and
wherein the access to the electronic healthcare records of the user is provided using a second mode of communication that is different from the first mode of communication, and further wherein the access to the electronic healthcare records of the user is provided from an aggregator (210), wherein, the electronic authorization enables the aggregator (210) to deliver a combined individual electronic healthcare record of the user sourced from the plurality of EHR systems to the provider device (604).

12. The mobile computing device (602) of claim 11, further comprising:
a display configured to display an image, wherein in the first mode of communication information is transmitted in the image,
wherein the image is displayed by the mobile computing device (602) for capture by a camera of the provider device (604), and wherein the image includes encoded information identifying the user of the mobile computing device (602), and an address of the electronic healthcare records of the user, and
wherein the image comprises a quick response code or a barcode that includes cryptographic keys and is displayed by the mobile computing device (602) for capture by a camera of the provider device (604), and wherein the provider device (604) is configured to use the cryptographic keys to access the electronic healthcare records of the user.

13. The mobile computing device (602) of claim 11, further comprising one or more radio frequency transceivers configured to:
transmit the electronic healthcare records of the user from the mobile computing device (602) to the provider device (604).

14. The mobile computing device (602) of claim 11, wherein one or more code sets are maintained by the mobile computing device (602), wherein the at least one processing circuit is configured to:
enable the user to parse, decode, aggregate, classify or organize the electronic healthcare records of the user by category based on the one or more code sets,
wherein the code sets include international or national standardized health data code lists for medications, diagnoses, laboratory tests, procedures, immunizations, or individual medical professions.

15. The mobile computing device (602) of claim 11, wherein the at least one processing circuit is configured to:
display electronic healthcare records in a language spoken by a healthcare professional using the provider device (604);
enable the healthcare professional to update or manipulate the electronic healthcare records using a spoken language of the healthcare professional;
determine a location of the mobile computing device (602) using location information derived using a global positioning satellite; and
automatically determine the language spoken by the healthcare professional using the location information.

## Patentansprüche

1. Verfahren zur Steuerung des Zugriffs auf elektronische Gesundheitsakten, wobei das Verfahren
umfasst:
Übertragen einer Unterstützungsanfrage von einer mobilen Vorrichtung an eine Anbietervorrichtung unter Verwendung eines ersten Kommunikationsmodus, wobei die Unterstützungsanfrage als Reaktion auf einen medizinischen Zustand eines Benutzers erzeugt wird, der von der mobilen Vorrichtung erkannt wird;
als Reaktion auf die Unterstützungsanfrage, Empfangen von Informationen durch die mobile Vorrichtung, die eine Identität der Anbietervorrichtung authentifizieren; und
Bereitstellen einer elektronischen Autorisierung durch die mobile Vorrichtung an die Anbietervorrichtung, wenn die Identität der Anbietervorrichtung authentifiziert wurde, wobei die elektronische Autorisierung die Zustimmung des Benutzers für die Anbietervorrichtung zum Zugriff auf elektronische Gesundheitsakten des Benutzers aus einer Vielzahl von elektronischen Patientenakten-, EHR-, Systemen bereitstellt, die von mindestens zwei verschiedenen Anbietern geführt werden;
wobei die elektronische Autorisierung der Anbietervorrichtung Zugriff auf elektronische Gesundheitsakten des Benutzers der mobilen Vorrichtung bereitstellt, und
wobei der Zugriff auf die elektronischen Gesundheitsakten des Benutzers unter Verwendung eines zweiten Kommunikationsmodus bereitgestellt wird, der sich von dem ersten Kommunikationsmodus unterscheidet, und weiter, wobei der Zugriff auf die elektronischen Gesundheitsakten des Benutzers von einem Aggregator bereitgestellt wird, wobei
die elektronische Autorisierung es dem Aggregator ermöglicht, eine kombinierte individuelle elektronische Gesundheitsakte des Benutzers aus der Vielzahl von EHR-Systemen an die Anbietervorrichtung bereitzustellen.

2. Verfahren nach Anspruch 1, wobei der erste Kommunikationsmodus von der mobilen Vorrichtung verwendet wird, um ein Bild zwischen der mobilen Vorrichtung und der Anbietervorrichtung zu übertragen.

3. Verfahren nach Anspruch 2, wobei das Bild von der mobilen Vorrichtung zur Erfassung durch eine Kamera der Anbietervorrichtung angezeigt wird, und wobei das Bild verschlüsselte Informationen zur Identifizierung des Benutzers der mobilen Vorrichtung und Adressinformationen, die zum Zugriff auf die elektronischen Gesundheitsakten des Benutzers verwendet werden, einschließt.

4. Verfahren nach Anspruch 2, wobei das Bild einen Schnellantwortcode oder einen Barcode umfasst, der kryptographische Schlüssel einschließt und von der mobilen Vorrichtung zur Erfassung durch eine Kamera der Anbietervorrichtung angezeigt wird, und wobei die Anbietervorrichtung so ausgebildet ist, dass sie die kryptographischen Schlüssel zum Zugriff auf die elektronischen Gesundheitsakten des Benutzers verwendet.

5. Verfahren nach Anspruch 1, weiter umfassend:
Übertragen der elektronischen Autorisierung an einen Netzwerkspeicher bestimmter elektronischer Gesundheitsakten des Benutzers, die von dem Netzwerkspeicher geführt werden,
wobei die elektronische Autorisierung als Zustimmung zum Übertragen der elektronischen Gesundheitsakten des Benutzers aus dem Netzwerkspeicher an die Anbietervorrichtung dient.

6. Verfahren nach Anspruch 5, weiter umfassend:
Abrufen bestimmter elektronischer Gesundheitsakten des Benutzers aus dem Netzwerkspeicher und Übertragen der elektronischen Gesundheitsakten des Benutzers an die Anbietervorrichtung.

7. Verfahren nach Anspruch 1, wobei ein oder mehrere Codesätze, die eine internationale oder nationale standardisierte Gesundheitsdatencodeliste einschließen, von der mobilen Vorrichtung geführt werden, und weiter umfassend:
Ermächtigen eines Benutzers, die elektronischen Gesundheitsakten des Benutzers auf Grundlage eines oder mehrerer Codesätze zu analysieren, zu entschlüsseln, zu aggregieren, zu klassifizieren oder nach Kategorien zu ordnen.

8. Verfahren nach Anspruch 7, wobei die Codesätze internationale oder nationale standardisierte Gesundheitsdatencodelisten für Medikamente, Diagnosen, Labortests, Verfahren, Impfungen oder einzelne medizinische Berufe einschließen.

9. Verfahren nach Anspruch 7, wobei elektronische Gesundheitsakten in einer Sprache angezeigt, aktualisiert oder manipuliert werden, die von einer medizinischen Fachkraft unter Verwendung der elektronischen Gesundheitsakten gesprochen wird.

10. Verfahren nach Anspruch 9, wobei die von der medizinischen Fachkraft gesprochene Sprache automatisch durch GPS-Ortung oder einen anderen Ortungsdienst bestimmt wird.

11. Mobile Computervorrichtung (602), die für drahtlose Kommunikation ausgebildet ist und Folgendes umfasst:
mindestens eine Verarbeitungsschaltung, wobei die mindestens eine Verarbeitungsschaltung für Folgendes ausgebildet ist:
Veranlassen des Sendens einer Unterstützungsanfrage von der mobilen Computervorrichtung (602) an eine Anbietervorrichtung (604) unter Verwendung eines ersten Kommunikationsmodus, wobei die Unterstützungsanfrage als Reaktion auf einen medizinischen Zustand eines Benutzers erzeugt wird, der von der mobilen Computervorrichtung (602) erkannt wird;
als Reaktion auf die Unterstützungsanfrage, Empfangen von Informationen zur Authentifizierung einer Identität der Anbietervorrichtung (604); und
Bereitstellen einer elektronischen Autorisierung für die Anbietervorrichtung (604), wenn die Identität der Anbietervorrichtung (604) authentifiziert wurde, wobei die elektronische Autorisierung die Zustimmung des Benutzers für die Anbietervorrichtung (604) zum Zugriff auf elektronische Gesundheitsakten des Benutzers aus einer Vielzahl von elektronischen Patientenakten-, EHR-, Systemen aufweist, die von mindestens zwei verschiedenen Anbietern geführt werden;
wobei die elektronische Autorisierung der Anbietervorrichtung (604) Zugriff auf elektronische Gesundheitsakten des Benutzers der mobilen Computervorrichtung (602) bereitstellt, und
wobei der Zugriff auf die elektronischen Gesundheitsakten des Benutzers unter Verwendung eines zweiten Kommunikationsmodus bereitgestellt wird, der sich von dem ersten Kommunikationsmodus unterscheidet, und
weiter, wobei der Zugriff auf die elektronischen Gesundheitsakten des Benutzers von einem Aggregator (210) bereitgestellt wird, wobei die elektronische Autorisierung es dem Aggregator (210) ermöglicht, eine kombinierte individuelle elektronische Gesundheitsakte des Benutzers aus der Vielzahl von EHR-Systemen an die Anbietervorrichtung (604) bereitzustellen.

12. Mobile Computervorrichtung (602) nach Anspruch 11, weiter umfassend:
eine Anzeige, die zur Anzeige eines Bildes ausgebildet ist, wobei in dem ersten Modus Kommunikationsinformationen in dem Bild übertragen werden,
wobei das Bild von der mobilen Computervorrichtung (602) zur Erfassung durch eine Kamera der Anbietervorrichtung (604) angezeigt wird, und wobei das Bild verschlüsselte Informationen zur Identifizierung des Benutzers der mobilen Computervorrichtung (602) und eine Adresse der elektronischen Gesundheitsakten des Benutzers einschließt, und
wobei das Bild einen Schnellantwortcode oder einen Barcode umfasst, der kryptographische Schlüssel einschließt und von der mobilen Computervorrichtung (602) zur Erfassung durch eine Kamera der Anbietervorrichtung (604) angezeigt wird und wobei die Anbietervorrichtung (604) so ausgebildet ist, dass sie die kryptographischen Schlüssel zum Zugriff auf die elektronischen Gesundheitsakten des Benutzers verwendet.

13. Mobile Computervorrichtung (602) nach Anspruch 11, weiter umfassend einen oder mehrere Funkfrequenz-Transceiver, die für Folgendes ausgebildet sind:
Übertragen der elektronischen Gesundheitsakten des Benutzers von der mobilen Computervorrichtung (602) an die Anbietervorrichtung (604).

14. Mobile Computervorrichtung (602) nach Anspruch 11, wobei ein oder mehrere Codesätze von der mobilen Computervorrichtung (602) geführt werden, wobei die mindestens eine Verarbeitungsschaltung für Folgendes ausgebildet ist:
Ermächtigen des Benutzers, die elektronischen Gesundheitsakten des Benutzers auf Grundlage eines oder mehrerer Codesätze nach Kategorien zu analysieren, zu entschlüsseln, zu aggregieren, zu klassifizieren oder zu organisieren, wobei die Codesätze internationale oder nationale standardisierte Gesundheitsdaten-Codelisten für Medikamente, Diagnosen, Labortests, Verfahren, Impfungen oder einzelne medizinische Berufe einschließen.

15. Mobile Computervorrichtung (602) nach Anspruch 11, wobei die mindestens eine Verarbeitungsschaltung für Folgendes ausgebildet ist:
Anzeigen elektronischer Gesundheitsakten in einer Sprache, die von einer medizinischen Fachkraft gesprochen wird, unter Verwendung der Anbietervorrichtung (604);
Ermächtigen der medizinischen Fachkraft zur Aktualisierung oder Manipulation der elektronischen Gesundheitsakten unter Verwendung der gesprochenen Sprache der medizinischen Fachkraft;
Bestimmen einer Position der mobilen Computervorrichtung (602) unter Verwendung von Positionsinformationen, die mithilfe eines globalen Positionierungssatelliten abgeleitet werden; und
automatisches Bestimmen der von der medizinischen Fachkraft gesprochenen Sprache anhand der Standortinformationen.

## Revendications

1. Procédé de commande d'accès à des dossiers médicaux électroniques, le procédé comprenant :
la transmission d'une demande d'assistance d'un dispositif mobile à un dispositif fournisseur en utilisant un premier mode de communication, dans lequel la demande d'assistance est générée en réponse à une affection médicale d'un utilisateur qui est détectée par le dispositif mobile ;
en réponse à la demande d'assistance, la réception par le dispositif mobile d'informations authentifiant une identité du dispositif fournisseur ; et
la fourniture par le dispositif mobile d'une autorisation électronique au dispositif fournisseur lorsque l'identité du dispositif fournisseur a été authentifiée, l'autorisation électronique prouvant le consentement de l'utilisateur pour que le dispositif fournisseur accède aux dossiers médicaux électroniques de l'utilisateur à partir d'une pluralité de systèmes de dossiers médicaux électroniques, DME, conservés par au moins deux dispositifs fournisseurs différents ;
dans lequel l'autorisation électronique fournit au dispositif fournisseur un accès aux dossiers médicaux électroniques de l'utilisateur du dispositif mobile, et
dans lequel l'accès aux dossiers médicaux électroniques de l'utilisateur est fourni en utilisant un second mode de communication qui est différent du premier mode de communication, et en outre dans lequel l'accès aux dossiers médicaux électroniques de l'utilisateur est fourni à partir d'un agrégateur, dans lequel, l'autorisation électronique permet à l'agrégateur de délivrer un dossier médical électronique individuel combiné de l'utilisateur provenant de la pluralité de systèmes DME au dispositif fournisseur.

2. Procédé selon la revendication 1, dans lequel le premier mode de communication est utilisé par le dispositif mobile pour transférer une image entre le dispositif mobile et le dispositif fournisseur.

3. Procédé selon la revendication 2, dans lequel l'image est affichée par le dispositif mobile pour être capturée par une caméra du dispositif fournisseur, et dans lequel l'image inclut des informations codées identifiant l'utilisateur du dispositif mobile, et des informations d'adresse utilisées pour accéder aux dossiers médicaux électroniques de l'utilisateur.

4. Procédé selon la revendication 2, dans lequel l'image comprend un code de réponse rapide ou un code-barres qui inclut des clés cryptographiques et est affiché par le dispositif mobile pour être capturé par une caméra du dispositif fournisseur, et dans lequel le dispositif fournisseur est configuré pour utiliser les clés cryptographiques pour accéder aux dossiers médicaux électroniques de l'utilisateur.

5. Procédé selon la revendication 1, comprenant en outre :
la transmission de l'autorisation électronique à un référentiel réseau de certains dossiers médicaux électroniques de l'utilisateur conservés par le référentiel réseau,
dans lequel l'autorisation électronique sert de consentement pour transmettre les dossiers médicaux électroniques de l'utilisateur du référentiel de réseau au dispositif fournisseur.

6. Procédé selon la revendication 5, comprenant en outre :
la récupération de certains dossiers médicaux électroniques de l'utilisateur à partir du référentiel de réseau ; et la transmission des dossiers médicaux électroniques de l'utilisateur au dispositif fournisseur.

7. Procédé selon la revendication 1, dans lequel un ou plusieurs ensembles de codes qui incluent une liste de codes de données de santé normalisées internationales ou nationales sont conservés par le dispositif mobile, et comprenant en outre :
la permission à un utilisateur d'analyser, de décoder, d'agréger, de classer ou d'organiser les dossiers médicaux électroniques de l'utilisateur par catégorie sur la base des un ou plusieurs ensembles de codes.

8. Procédé selon la revendication 7, dans lequel les ensembles de codes incluent des listes de codes de données de santé normalisées internationales ou nationales pour des médicaments, des diagnostics, des tests de laboratoire, des procédures, des immunisations ou des professions médicales individuelles.

9. Procédé selon la revendication 7, dans lequel les dossiers médicaux électroniques sont affichés, mis à jour ou manipulés dans une langue parlée par un professionnel de santé utilisant les dossiers médicaux électroniques.

10. Procédé selon la revendication 9, dans lequel la langue parlée par le professionnel de santé est déterminée automatiquement par la localisation GPS ou un autre service de localisation.

11. Dispositif informatique mobile (602) configuré pour des communications sans fil et comprenant :
au moins un circuit de traitement, dans lequel l'au moins un circuit de traitement est configuré pour : provoquer la transmission d'une demande d'assistance du dispositif informatique mobile (602) à un dispositif fournisseur (604) en utilisant un premier mode de communication, dans lequel la demande d'assistance est générée en réponse à une affection médicale d'un utilisateur qui est détectée par le dispositif informatique mobile (602) ;
recevoir, en réponse à la demande d'assistance, des informations authentifiant une identité du dispositif fournisseur (604) ; et
fournir une autorisation électronique au dispositif fournisseur (604) lorsque l'identité du dispositif fournisseur (604) a été authentifiée, l'autorisation électronique prouvant le consentement de l'utilisateur pour que le dispositif fournisseur (604) accède aux dossiers médicaux électroniques de l'utilisateur à partir d'une pluralité de systèmes de dossiers médicaux électroniques, DME, conservés par au moins deux fournisseurs différents ;
dans lequel l'autorisation électronique fournit au dispositif fournisseur (604) un accès aux dossiers médicaux électroniques de l'utilisateur du dispositif informatique mobile (602), et
dans lequel l'accès aux dossiers médicaux électroniques de l'utilisateur est fourni en utilisant un second mode de communication qui est différent du premier mode de communication, et en outre dans lequel l'accès aux dossiers médicaux électroniques de l'utilisateur est fourni à partir d'un agrégateur (210), dans lequel l'autorisation électronique permet à l'agrégateur (210) de délivrer un dossier médical électronique individuel combiné de l'utilisateur provenant de la pluralité de systèmes DME au dispositif fournisseur (604).

12. Dispositif informatique mobile (602) selon la revendication 11, comprenant en outre :
un dispositif d'affichage configuré pour afficher une image, dans lequel dans le premier mode de communication, des informations sont transmises dans l'image,
dans lequel l'image est affichée par le dispositif informatique mobile (602) pour être capturée par une caméra du dispositif fournisseur (604), et dans lequel l'image inclut des informations codées identifiant l'utilisateur du dispositif informatique mobile (602), et une adresse des dossiers médicaux électroniques de l'utilisateur, et
dans lequel l'image comprend un code de réponse rapide ou un code-barres qui inclut des clés cryptographiques et est affiché par le dispositif informatique mobile (602) pour être capturé par une caméra du dispositif fournisseur (604), et dans lequel le dispositif fournisseur (604) est configuré pour utiliser les clés cryptographiques pour accéder aux dossiers médicaux électroniques de l'utilisateur.

13. Dispositif informatique mobile (602) selon la revendication 11, comprenant en outre un ou plusieurs émetteurs-récepteurs radiofréquence configurés pour :
transmettre les dossiers médicaux électroniques de l'utilisateur du dispositif informatique mobile (602) au dispositif fournisseur (604).

14. Dispositif informatique mobile (602) selon la revendication 11, dans lequel un ou plusieurs ensembles de codes sont conservés par le dispositif informatique mobile (602), dans lequel l'au moins un circuit de traitement est configuré pour :
permettre à l'utilisateur d'analyser, de décoder, d'agréger, de classer ou d'organiser les dossiers médicaux électroniques de l'utilisateur par catégorie sur la base d'un ou plusieurs ensembles de codes, dans lequel les ensembles de codes incluent des listes de codes de données de santé normalisées internationales ou nationales pour des médicaments, des diagnostics, des tests de laboratoire, des procédures, des immunisations ou des professions médicales individuelles.

15. Dispositif informatique mobile (602) selon la revendication 11, dans lequel l'au moins un circuit de traitement est configuré pour :
afficher des dossiers médicaux électroniques dans une langue parlée par un professionnel de santé utilisant le dispositif fournisseur (604) ;
permettre au professionnel de santé de mettre à jour ou de manipuler les dossiers médicaux électroniques en utilisant une langue parlée du professionnel de santé ;
déterminer une localisation du dispositif informatique mobile (602) en utilisant des informations de localisation dérivées en utilisant un satellite de positionnement global ; et
déterminer automatiquement la langue parlée par le professionnel de santé en utilisant les informations de localisation.
